# EUROPEAN PATENT SPECIFICATION

(11) **EP 3 370 507 B1**
(45) Date of publication and mention of the grant of the patent: **09.12.2026**
(21) Application number: 16822634.8
(22) Date of filing: 15.12.2016
(51) Int. Cl.: A01H 1/04, A01H 5/10, C07K 14/415, C12Q 1/68, C12N 15/82

(54) **BRASSICACEAE PLANTS RESISTANT TO PLASMODIOPHORA BRASSICAE (CLUBROOT)**
GEGEN PLASMODIOPHORA BRASSICAE (KOHLHERNIE) RESISTENTE KREUZBLÜTLER
BRASSICACÉES RÉSISTANTES À PLASMODIOPHORA BRASSICAE (HERNIE)

(30) Priority: 15.12.2015 US 201562267460 P
(43) Date of publication of application: 12.09.2018
(73) Proprietor: BASF Agricultural Solutions US LLC, Research Triangle Park, NC 27713 (US)
(72) Inventor: ENGELEN, Steven, 9080 Zeveneken - Lochristi (BE); VAN THOURNOUT, Michel, 9052 Gent (BE); RAE, Stephen, 9052 Gent (BE); CROMMAR, Kim, 9052 Gent (BE); HOSTYN, Vanessa, 9052 Gent (BE); CHONGO, Godfrey, Saskatchewan S0K 4S0 (CA)
(74) Representative: AP-IP
(86) International application number: PCT/EP2016/081146
(87) International publication number: WO 2017/102923

(56) References cited:
- EP-A1- 2 745 679
- US-A1- 2013 254 929
- ZHONGYUN PIAO ET AL: "Genetics of Clubroot Resistance in Brassica Species", JOURNAL OF PLANT GROWTH REGULATION, SPRINGER-VERLAG, NE, vol. 28, no. 3, 16 April 2009 (2009-04-16), pages 252 - 264, XP019739174, ISSN: 1435-8107, DOI: 10.1007/S00344-009-9093-8
- "Plants for the Future", 21 October 2015, INTECH, ISBN: 978-953-51-2185-5, article ARVIND H. HIRANI ET AL: "Understanding the Genetics of Clubroot Resistance for Effectively Controlling this Disease in Brassica Species", XP055347529, DOI: 10.5772/60936
- ANONYMOUS: "UPI0006AA7366", 1 September 2015 (2015-09-01), XP055347928, Retrieved from the Internet <URL:http://www.uniprot.org/uniparc/UPI0006AA7366> [retrieved on 20170221]
- ANONYMOUS: "PREDICTED: uncharacterized protein LOC106400730 isoform X3 [Brassica n - Protein - NCBI", 31 August 2015 (2015-08-31), XP055347933, Retrieved from the Internet <URL:https://www.ncbi.nlm.nih.gov/protein/XP_013696576> [retrieved on 20170221]
- ANONYMOUS: "UPI0006AB5B72", 1 September 2015 (2015-09-01), XP055347938, Retrieved from the Internet <URL:http://www.uniprot.org/uniparc/UPI0006AB5B72> [retrieved on 20170221]
- ANONYMOUS: "PREDICTED: uncharacterized protein LOC106400730 isoform X1 [Brassica n - Protein - NCBI", 31 August 2015 (2015-08-31), XP055347939, Retrieved from the Internet <URL:https://www.ncbi.nlm.nih.gov/protein/XP_013696562> [retrieved on 20170221]
- ANONYMOUS: "UPI0006AB5F4F", 1 September 2015 (2015-09-01), XP055347941, Retrieved from the Internet <URL:http://www.uniprot.org/uniparc/UPI0006AB5F4F> [retrieved on 20170221]
- ANONYMOUS: "PREDICTED: uncharacterized protein LOC106400730 isoform X2 [Brassica n - Protein - NCBI", 31 August 2015 (2015-08-31), XP055347942, Retrieved from the Internet <URL:https://www.ncbi.nlm.nih.gov/protein/XP_013696569> [retrieved on 20170221]
- ANONYMOUS: "UPI0006AA8AFA", 1 September 2015 (2015-09-01), XP055347943, Retrieved from the Internet <URL:http://www.uniprot.org/uniparc/UPI0006AA8AFA> [retrieved on 20170221]
- ANONYMOUS: "PREDICTED: probable disease resistance protein At1g58390 isoform X4 [B - Protein - NCBI", 31 August 2015 (2015-08-31), XP055347945, Retrieved from the Internet <URL:https://www.ncbi.nlm.nih.gov/protein/XP_013696585> [retrieved on 20170221]
- ANONYMOUS: "UPI0004EE8C51", 29 October 2014 (2014-10-29), XP055347946, Retrieved from the Internet <URL:http://www.uniprot.org/uniparc/UPI0004EE8C51> [retrieved on 20170221]
- CHALHOUB B.,: "A0A078GQZ4", 29 October 2014 (2014-10-29), XP055347711, Retrieved from the Internet <URL:http://www.uniprot.org/uniprot/A0A078GQZ4.txt> [retrieved on 20170220]

## Description

### FIELD OF THE INVENTION

The invention relates to the field of disease control in Brassicaceae. Provided are methods for the production of clubroot resistant plants through introduction of a clubroot resistance locus in their genome. Also provided are Brassicaceae plants and seeds comprising one or more clubroot resistance loci in their genome. Further provided are detection tools for detecting the presence of one or more resistance alleles in Brassicaceae plants, tissue or seeds, as well as methods for transferring one or more resistance loci to other Brassicaceae plants and methods for combining different resistance loci in hybrid seeds and plants. Methods for enhancing durability of resistance to *Plasmodiophora brassicae* are also provided, as well as uses of the plants and seeds and the processes or kits of the invention.

### BACKGROUND OF THE INVENTION

Clubroot is a disease caused by *Plasmodiophora brassicae* which affects the Brassicaceae family of plants, including many important vegetable and broad acre crops. All members of the family Brassicaceae are thought to be potential hosts for *Plasmodiophora brassicae* (Dixon, 2009, J Plant Growth Regul 28: 194). Susceptible cultivated crops include all varieties of *B. oleracea,* the Occidental Cole vegetables (Brussels sprout, cabbages, calabrese/green broccoli, cauliflower, culinary and fodder kale, kohlrabi); *B. rapa* (syn. *B. campestris)* including turnip, turnip rape, sarson, and the enormous range of Oriental variants which provide leaf and root vegetables such as *Brassica rapa* var. *pekinensis* and *B. rapa* var. *chinensis* (Chinese cabbages); *B. napus* including swede (rutabaga), oil seed rape, and fodder rape; and seed, condiment (mustard), and vegetable crops derived from *B. carinata, B. nigra,* and *B. juncea.* Related genera such as radish *(Raphanus),* cruciferous weeds, for example, *Sinapis,* and decorative ornamentals including stocks *(Matthiola* spp) and wallflower *(Cheiranthus cheiri)* can be infected. The scientific model plant *Arabidopsis* is also susceptible (Dixon, 2009, supra).

Clubroot disease symptom development is characterized by the formation of club-shaped galls on the roots of affected plants. As a result, the nutrient and water uptake by infected roots is inhibited. Above-ground symptoms include wilting, stunting, yellowing and premature senescence (Hwang et al, 2012, Mol Plant Pathol 13: 105).

Clubroot disease is estimated to be present in approximately 10% of all areas where host plants are cultivated (Diederichsen et al, 2009, J Plant Growth Regul 28: 265). Clubroot has been largely a disease of vegetable crops in the last century. However, in 2003, 12 clubroot-infested commercial fields were found in the central part of the province of Alberta. Thereafter, the number of fields with confirmed clubroot infestations has increased steadily, and, by 2010, more than 560 fields (over 35 000 ha) in Alberta had been identified as being infested with *P. brassicae* (Hwang et al., 2012, supra). Yield losses of 80%-91% were reported in studies with canola grown on clubroot-infested fields in Quebec. Seed quality was also reduced significantly, with declines of 4.7%-6.1% in oil content and 13%-26% in 1000-seed weights (Hwang et al., 2012, supra).

Plant resistance is a powerful tool to combat clubroot disease. Breeding for clubroot resistance focuses today on Chinese cabbage *(B. rapa* spp. *Pekinensis)* in Japan and Korea, oilseed rape in Germany and Sweden, and several *B. oleracea* vegetables. Recently released resistant cultivars belong to three Brassica species: *B. napus, B. oleracea,* and *B. rapa* (Diederichsen et al., 2009, supra).

Resistant sources of the European fodder turnips *(B. rapa* ssp, *rapifera*) have been identified, which have been used to transfer the clubroot resistance genes to Chinese cabbage. At least three independent dominant genes, which confer differential (race-specific or vertical) resistance to particular pathotypes of *P. brassicae,* appear to be present in turnip genotypes (Piao et al., 2009, J Plant Growth Regul 28: 252). Eight possible clubroot resistance genes present in *B. rapa* have been identified through QTL mapping: *CRa* from resistant source ECD02, *CRb* from Gelria R, *Crr1, Crr2* and *Crr4* from Siloga, *Crr3* from Milan White, and *CRk* and *CRc* from Debra. *Crr1, Crr2, Crr3, Crr4* and *CRc* are mapped to chromosomes R8, R1, R3, R6 and R2, respectively. *CRa, CRb* and *CRk* with *Crr3* are mapped on the same linkage group of R3, but they are not located in the same chromosome region, except for *CRk* and *Crr3* (Piao et al., 2009, supra; Sakamoto et al., 2008, Theor Appl Genet 117:759).

In *B. oleracea,* completely resistant accessions have been rarely identified. The inheritance of the clubroot resistance in *B. oleracea* appears polygenic and controlled by many dominant alleles with predominance of additive effects of with incomplete dominance. It has also been suggested that one of the resistances studied is controlled by two complementary genes (Piao et al., 2009, supra). At least 22 QTLs have been found in *B. oleracea,* indicating a complex genetic basis of clubroot resistance in *B. oleracea.* As the different mapping studies used different clubroot resistance sources and different P. *brassicae* isolates, a comparison of these QTLs is not possible (Piao et al., 2009, supra).

Clubroot resistance has also been observed in several *B. napus* cultivars. At least 22 QTLs for clubroot resistance have been identified in *B. napus.* A major gene, Pb-Bn1, has been mapped onto linkage group DY4, and at least two additive QTLs have been identified on chromosomes DY4 and DY15, respectively. In addition, epistatic interactions between nine regions with or without additive effects have been located. A major gene and two recessive genes derived from ECD04 have been identified in double-haploid populations. In resynthesized B. napus developed by crossing cv. Böhmerwaldkohl *(B. oleracea)* and ECD-04 *(B. rapa),* nineteen QTLs expressing resistance to seven isolated were detected on eight chromosomes, four of which were closely linked to each other on chromosome N03, and three were linked on chromosome N08. Genes CRk and Crr3 are located in the similar region of *PbBn-k-2, PbBn-1-1,* and *PbBn-01:60-1* on N03. *CRa* and *CRb* are independent from them. *PbBn-01.07-2, PbBn-l-2,* and *PbBn-a-1* are linked to *BRMS088* on chromosome N08 in *B. napus,* which is also linked with *Crr1* on R8 in *B. rapa. PbBn-k-1* is located on chromosome N02. The QTLs located on N03 and N19 contribute strong effects and confer broad-spectrum resistance (Piao et al., 2009, supra; and Werner et al., 2008, Theor Appl Genet 116:363).

The *CRa* gene of *Brassica rapa* has been fine-mapped and a TIR-NBS-LRR gene has been identified as the CRa gene (Ueno et al., 2012, Plant Mol Biol 80: 621). The Crr1 gene has been mapped and isolated from the *B. rapa* European fodder turnip "Siloga". *Crr1a* also encodes a TIR-NB-LRR disease resistance protein (Hatakeyama et al., 2013, PLOS one 8: e54745 and WO2012/039445).

The *CRb* gene from *B. rapa* has been fine-mapped to a 140 kb genomic region. In this region, in which fourteen functional proteins were predicted, amongst which a Rho family proteins and two TIR-NBS-LRR proteins, which could be candidate genes for CRb (Kato et al., 2013, Breeding Science 63: 116).

To increase the durability of clubroot-resistant cultivars, the combination of the different clubroot resistance genes into a single line will be an important means for breeding cultivars with resistance to a broader spectrum of physiological races. Therefore, in order to stack genes without linkage drag using marker-assisted selection and transgenic approaches, there remains a need to develop molecular markers linked to the clubroot resistance genes. This invention provides the sequence of a clubroot resistance locus from a resistant *Brassica napus* line, as herein after described in the different embodiments, examples and claims.

### SUMMARY OF THE INVENTION

It is one embodiment of the invention to provide a Brassicaceae plant or plant cell comprising a *CRL1* and a *CRL2* clubroot resistance gene as transgene, wherein said *CRL1* clubroot resistance gene comprises a coding sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or encodes a protein having an amino acid sequence having at least 90% sequence identity to SEQ ID NO: 3, SEQ ID NO: 5, SEQ ID NO: 7, or SEQ ID NO: 11; and said *CRL2* clubroot resistance gene comprises a coding sequence having at least 90% sequence identity to nt 220-2898 of SEQ ID NO: 8; or encodes a protein having an amino acid sequence having at least 90% sequence identity to SEQ ID NO: 9. In a further embodiment, said *CRL*1 clubroot resistance gene comprises a sequence having at least 90% sequence identity to nt 32750-51049 of SEQ ID NO: 1. In another embodiment, said Brassicaceae plant or plant cell comprises a transgene comprising a sequence having at least 90% sequence identity to SEQ ID NO: 1.

It is another object of the invention to provide a method for increasing clubroot resistance in a Brassicaceae plant, said method comprising introducing or providing a *CRL1* and a *CRL2* clubroot resistance gene according to the invention as a transgene , to a Brassicaceae plant cell, to create transgenic cells; and regenerating transgenic plants from said transgenic cells.

In yet another embodiment, a method is provided for increasing clubroot resistance in a Brassicaceae plant, comprising the step of introducing a *CRL* clubroot resistance locus in said Brassicaceae plant, and selecting said clubroot resistant Brassicaceae plant for the presence of the *CRL* clubroot resistance locus by analyzing genomic DNA from said plant for the presence of at least one molecular marker, wherein said at least one molecular marker is linked to the *CRL* clubroot resistance locus, wherein said *CRL* clubroot resistance locus comprises the *CRL1* and *CRL2* clubroot resistance genes, wherein said *CRL*1 clubroot resistance gene comprises a nucleotide sequence having at least 90% sequence identity to nt 32750 to 51049 of SEQ ID NO: 1; having a coding sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or encoding a protein having at least 90% sequence identity to SEQ ID NO: 3, SEQ ID NO: 5, SEQ ID NO: 7, or SEQ ID NO: 11; and wherein said *CRL2* clubroot resistance gene comprises a nucleotide sequence having a coding sequence having at least 90% sequence identity to nt 220-2898 of SEQ ID NO: 8; or encoding a protein having at least 90% sequence identity to SEQ ID NO: 9, such as a clubroot resistance locus comprising a sequence having at least 90% sequence identity to SEQ ID NO: 1.

Yet another embodiment provides a method for producing a clubroot resistant Brassicaceae plant comprising the steps of identifying a clubroot resistant Brassicaceae plant comprising a *CRL* clubroot resistance locus according to the invention by analyzing genomic DNA from said plant for the presence of at least one molecular marker, wherein said at least one molecular marker is linked to said *CRL* clubroot resistance locus, and generating progeny from said clubroot resistant Brassicaceae plant, wherein said progeny is clubroot resistant and comprises said *CRL* clubroot resistance locus. In a further embodiment, said *CRL* clubroot resistance locus comprises a sequence having at least 90% sequence identity to SEQ ID NO: 1.

A further embodiment provides Brassicaceae plants or plant cells obtainable by the methods according to the invention.

In a further embodiment, a clubroot resistant Brassicaceae plant or plant cell according to the invention is provided, comprising the *CRL1* and *CRL2* clubroot resistance genes according to the invention, and at least one other disease resistance gene, said other disease resistance gene selected from the group consisting of a clubroot resistance gene, a blackleg resistance gene, a Sclerotinia resistance gene, a Verticillium resistance gene, a Fusarium resistance gene, an Aster Yellows resistance gene, an Alternaria resistance gene, and a Grey Stem resistance gene. In a further embodiment, said other disease resistance gene is a transgene which is genetically linked with said *CRL* clubroot resistance genes or said *CRL* clubroot resistance locus.

In yet another embodiment, the plant according to the invention is selected from the group consisting of *Brassica napus, Brassica juncea, Brassica oleracea, Brassica rapa, Brassica nigra* and *Brassica carinata.* In yet another embodiment, seeds of the plants according to the invention are provided comprising the *CRL1* and *CRL2* clubroot resistance genes according to the invention.

A further embodiment provides methods to determine the presence or absence of a *CRL* clubroot resistance locus in a biological sample, comprising providing genomic DNA from said biological sample, and analyzing said DNA for the presence of at least one molecular marker, wherein the at least one molecular marker is linked to said *CRL* clubroot resistance locus, wherein said *CRL* clubroot resistance locus comprises the *CRL1* and *CRL2* clubroot resistance genes, wherein said *CRL1* clubroot resistance gene comprises a nucleotide sequence having at least 90% sequence identity to nt 32750-51049 of SEQ ID NO: 1; having a coding sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or encoding a protein having at least 90% sequence identity to SEQ ID NO: 3, SEQ ID NO: 5, SEQ ID NO: 7, or SEQ ID NO: 11; and wherein said *CRL2* clubroot resistance gene comprises a nucleotide sequence having a coding sequence having at least 90% sequence identity to nt 220-2898 of SEQ ID NO: 8; or encoding a protein having at least 90% sequence identity to SEQ ID NO: 9. In yet a further embodiment, a kit is provided for the detection of the *CRL* clubroot resistance locus according to the invention in Brassicaceae DNA samples, wherein said kit comprises at least one primer or probe which specifically recognizes a molecular marker linked to said *CRL* clubroot resistance locus. Yet another embodiment provides the use of a molecular marker linked to the *CRL* clubroot resistance locus according to the invention for determining the presence or absence of said *CRL* clubroot resistance locus in Brassicaceae plants, or the use of the sequence of any one of SEQ ID NOs: 1, 2, 4, 6 and 8 for determining the presence or absence of the *CRL* clubroot resistance locus according to the invention in Brassicaceae plants.

It is another object of the invention to provide a chimeric gene comprising the following genetic elements: a plant-expressible promoter, a DNA sequence coding for a CRL1 or for a CRL2 protein, and optionally, a transcription termination and polyadenylation region functional in plant cells, wherein said DNA sequence coding for a CRL1 protein comprises a nucleotide sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or encoding a protein having an amino acid sequence having at least 90% sequence identity to ID NO: 3, SEQ ID NO: 5, SEQ ID NO: 7, or SEQ ID NO: 11; and wherein said DNA sequence coding for a of CRL2 protein comprises a nucleotide sequence having at least 90% sequence identity to 220-2898 of SEQ ID NO: 8; or encoding a protein having an amino acid sequence having at least 90% sequence identity to SEQ ID NO: 9.

In another embodiment of the invention, the marker linked to the *CRL* clubroot resistance locus is located in a marker interval between and including markers mBRS00013411 and mBRS00013511, such as a marker selected from the markers of Table 2.

A further object provides the use of the chimeric gene according to the invention to increase clubroot resistance in Brassicaceae, and the use of the plants or plant cells according to the invention to produce oilseed rape oil or an oilseed rape seed cake, or a seed, or a crop of oilseed rape.

Also provided is a method of producing food, feed, or an industrial product, comprising obtaining the plant according to the invention or a part thereof; and preparing the food, feed or industrial product from the plant or part thereof. In a further object, said food or feed is oil, meal, grain, starch, flour or protein; or said industrial product is biofuel, fiber, industrial chemicals, a pharmaceutical or a nutraceutical.

### BRIEF DESCRIPTION OF THE DRAWINGS

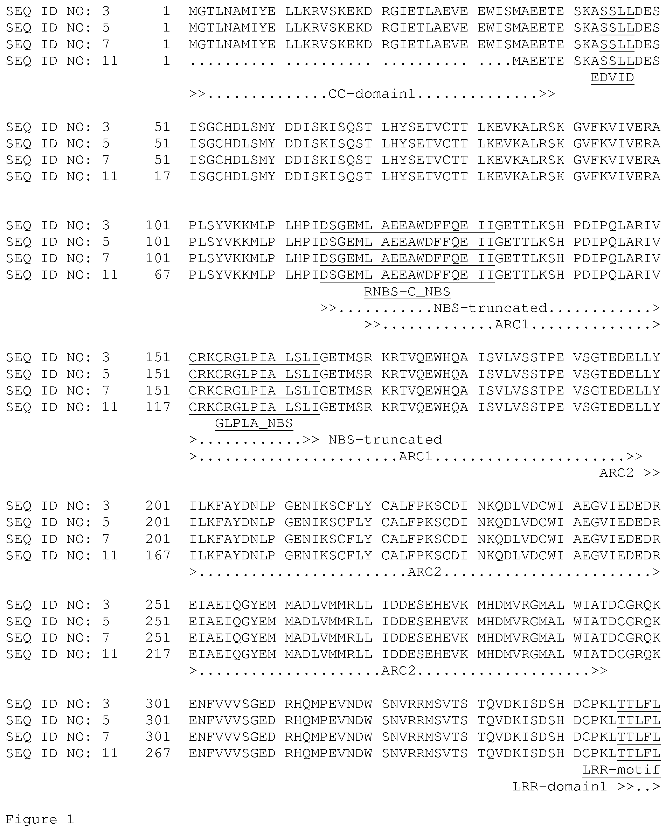

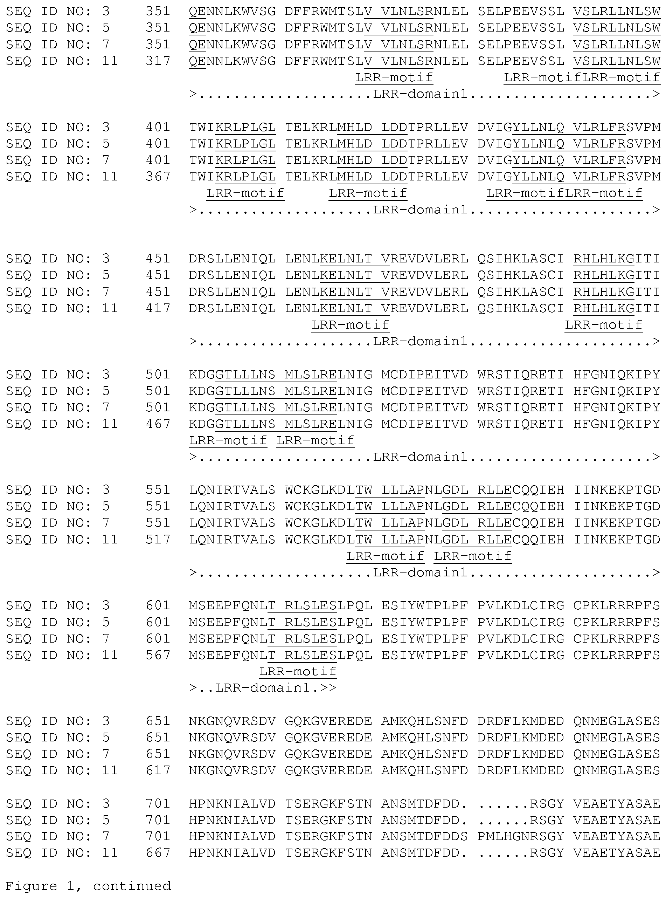

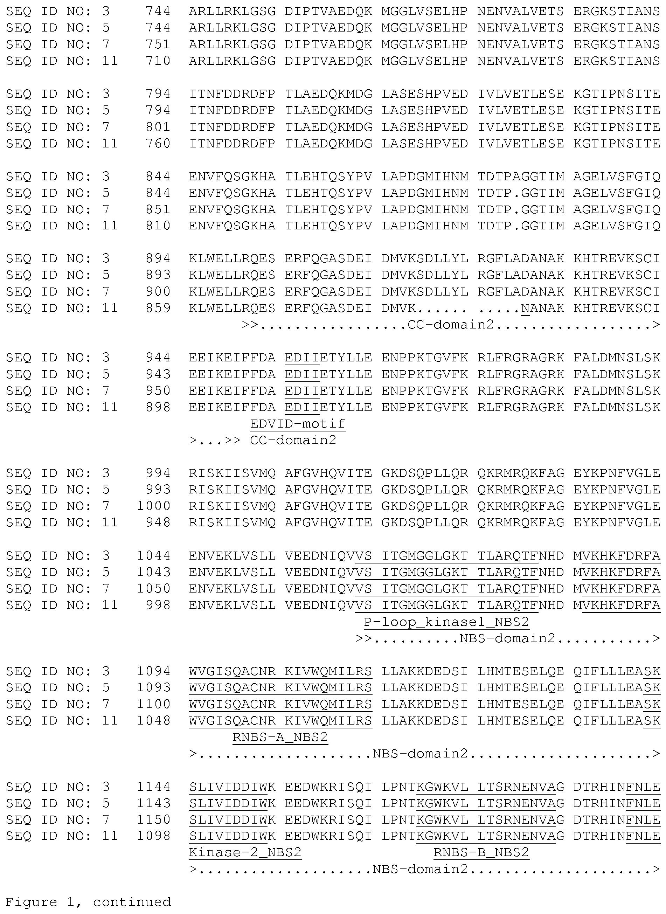

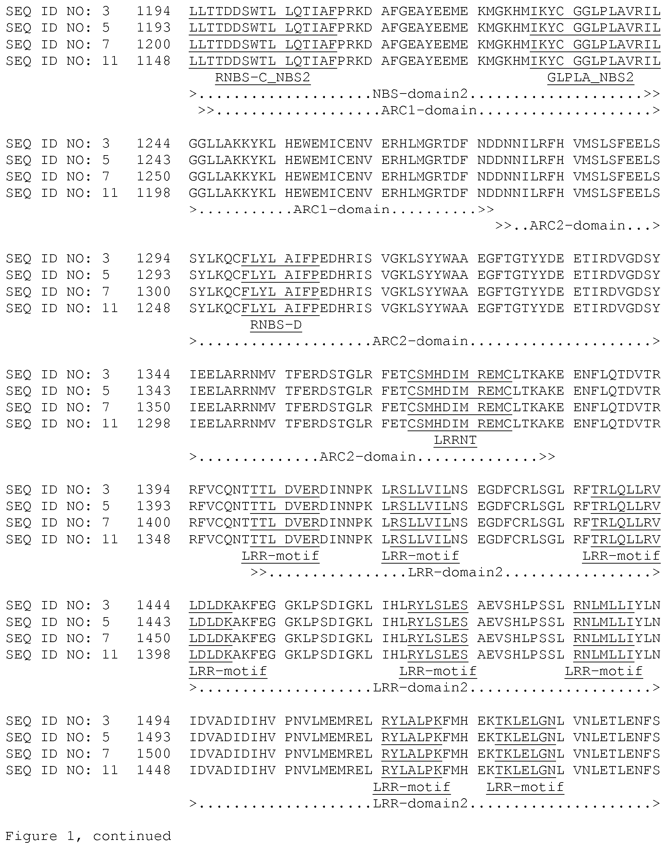

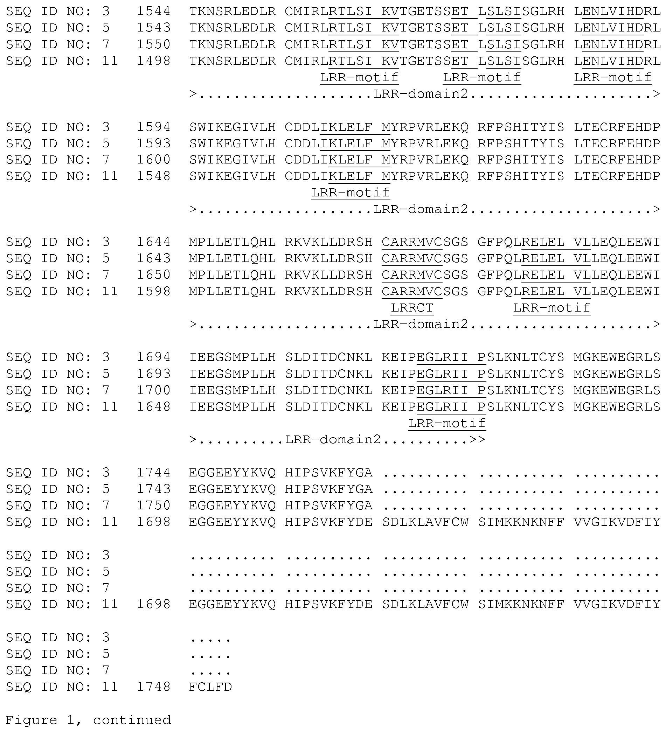

**Figure 1****:** Alignment of proteins encoded by the three different splice variants of CRL1: SEQ ID NO: 3 encoded by the *CRL*1a splice variant; SEQ ID NO: 5 encoded by the *CRL*1b splice variant, SEQ ID NO: 7 encoded by the *CRL*1c splice variant, and SEQ ID NO: 11 encoded by the *CRL*1d splice variant. The conserved domains are indicated. CC-domain1 = first coil-coil domain; EDVID: EDVID motif; NBS-truncated: truncated NBS domain with missing N-terminal part; RNBS-C NBS: RNBS-C region from the NBS domain; ARC1: ARC1 domain; GLPLA NBS: GLPLA or hydrophobic region of the NBS domain; ARC2: ARC2 domain; LRR-domain1: first Leucine Rich Region; LRR motif: Leucine rich repeat motif (xxLxLxx), CC-domain2: Second coil-coil domain; NBS-domain2: second NBS domain; P-loop kinase1 NBS2: P-loop/kinase 1 from the second NBS domain; RNBS-A_NBS2: RNBS-A region from the second NBS domain; Kinase-2_NBS2: Kinase 2 region of the second NBS domain; RNBS-B_NBS2: RNBS-B region from the second NBS domain. Also called Kinase3a or Sensor I; RNBS-C_NBS2: RNBS-C region from the second NBS domain: GLPLA_NBS2: GLPLA or hydrophobic region of the second NBS domain; RNBS-D: RNBS-D region; LRRNT: N-terminal cap - also known as MHD-region; LRR-domain2: second Leucine Rich Region; LRRCT: C-terminal cap.

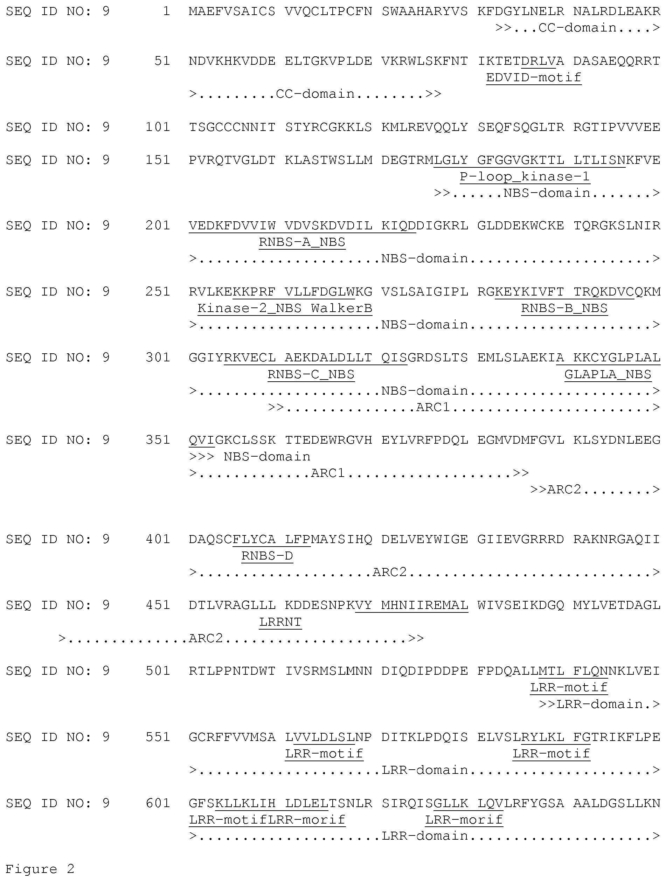

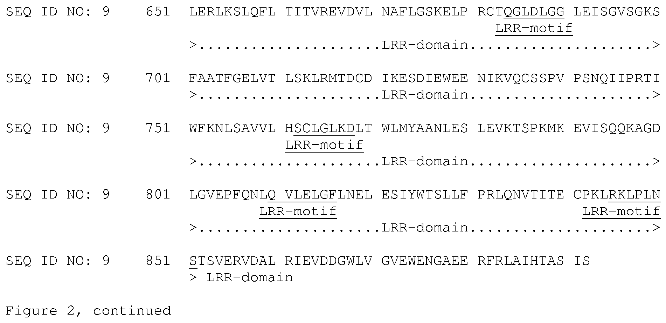

**Figure 2****:** CRL2 protein (SEQ ID NO: 9) and conserved domains. CC-domain: Coil-coil domain; P-loop_kinase-1: P-loop/kinase 1 from the NBS domain, also known as Walker A; RNBS-A_NBS: RNBS-A region from the NBS domain; Kinase-2_NBS: Kinase 2 region of the NBS domain; WalkerB: Walker B region; RNBS-B_NBS: RNBS-B region from the NBS domain. Also called Kinase 3a or Sensor I; RNBS-C_NBS: RNBS-C region from the NBS domain; GLAPLA_NBS: GLPLA or hydrophobic region of the NBS domain; RNBS-D: RNBS-D region; LRRNT: N-terminal cap - also known as MHD-region; LRR-domain: Leucine Rich Region; LRR-motif: Leucine rich repeat motif (xxLxLxx).

### DETAILED DESCRIPTION

The current invention is based on the identification of a *CRL* clubroot resistance locus in *Brassica.* Surprisingly, it was found that the *CRL* clubroot resistance locus as identified contains two *CRL* clubroot resistance genes.

The invention relates to sequences of the *CRL* clubroot resistance locus, and sequences encoding a protein conferring resistance to clubroot resistance in Brassicaceae. The protein may comprise the amino acid sequence having at least 80% sequence identity to SEQ ID NO: 3, to SEQ ID NO: 5, to SEQ ID NO: 7, or to SEQ ID NO: 9, or a functional fragment of these amino acid sequences.

It is a first embodiment of the invention to provide a Brassicaceae plant or plant cell comprising a *CRL1* and a *CRL2* clubroot resistance gene as transgene, wherein said *CRL1* clubroot resistance gene comprises a coding sequence having at least 90% sequence identity to nt 52-5343 of SEQ ID NO: 2, nt 52-5340 of SEQ ID NO: 4, nt 52-5361 of SEQ ID NO: 6 or to SEQ ID NO: 10; or encodes a protein having an amino acid sequence having at least 90% sequence identity to SEQ ID NO: 3, SEQ ID NO: 5, SEQ ID NO: 7, ro SEQ ID NO: 11; and said *CRL2* clubroot resistance gene comprises a coding sequence having at least 90% sequence identity to nt 220-2898 of SEQ ID NO: 8; or encodes a protein having an amino acid sequence having at least 90% sequence identity to SEQ ID NO: 9. In a further embodiment, said *CRL*1 clubroot resistance gene comprises a sequence having at least 90% sequence identity to nt 32750-51049 of SEQ ID NO: 1. In another embodiment, said Brassicaceae plant or plant cell comprises a transgene comprising a sequence having at least 90% sequence identity to SEQ ID NO: 1. Said transgene may also comprise a sequence having least 80%, or at least 85%, or at least 90%, or at least 92%, or at least 95%, or at least 98%, or at least 99%, or 100% sequence identity to nt 3145-51049 'of SEQ ID NO: 1, or to the sequence of SEQ ID NO: 1 without transposon regions, i.e. to the contiguous sequence of nt 3145-8010, 22965-30182, 30297-32257, 32645-45078 and 47953-51049 of SEQ ID NO: 1.

A "CRL protein", as used herein, is a protein encoded by a *CRL* clubroot resistance gene. A CRL protein can have an amino acid sequence having at least 80%, or at least 85%, or at least 90%, or at least 92%, or at least 95%, or at least 98%, or at least 99%, or 100% sequence identity to SEQ ID NO: 3, to SEQ ID NO: 5, to SEQ ID NO: 7, or to SEQ ID NO: 9.

A "functional fragment" of the amino acid sequence having at least 80% sequence identity to SEQ ID NO: 3, denotes a protein or peptide comprising a stretch of the amino acid sequences as depicted above which still exerts the desired function, i.e. which increases clubroot resistance when present in a Brassicaceae plant. An assay for determining of whether a functional fragment increases clubroot resistance is provided herein.

*A "CLR* clubroot resistance gene", or *"CRL* resistance gene", or *"CRL* gene", as used herein, is a gene that confers resistance to *Plasmodiophora brassicae* strain Leduc-ss2 corresponding to pathotype 6 as described by Xue et al., 2008, Plant Disease 92:456 (herein incorporated by reference). A *CRL* resistance gene is present, for example, in *Brassica napus* cvs. Laurentian, Nevin and Wilhelmsburger or, for example, in *Brassica oleracea* cv. Badger Shipper (see Xue et al, 2008, supra). A *"CRL* clubroot resistance gene", *"CRL* resistance gene" or *"CRL* gene" can be sufficient for resistance to *Plasmodiophora brassicae* strain Leduc-ss2 corresponding to pathotype 6. A *"CRL* clubroot resistance gene" or *"CRL* gene" can also be required together with another *CRL* clubroot resistance gene for resistance to *Plasmodiophora brassicae* strain Leduc-ss2 corresponding to pathotype 6.

A *CRL* clubroot resistance gene, or *CRL* gene can encode a CRL amino acid sequence having at least 80%, or at least 85%, or at least 90%, or at least 92%, or at least 95%, or at least 98%, or at least 99%, or 100% sequence identity to SEQ ID NO: 3, or to SEQ ID NO: 5, or to SEQ ID NO: 7, or to SEQ ID NO: 9. A *CRL* clubroot resistance gene, or *CRL* gene, can comprise a nucleotide sequence having at least 80%, or at least 85%, or at least 90%, or at least 92%, or at least 95%, or at least 98%, or at least 99%, or 100% sequence identity to SEQ ID NO: 2, or to SEQ ID NO: 4, or to SEQ ID NO: 6, to SEQ ID NO: 8, to SEQ ID NO: 10, to nt 52-5343 of SEQ ID NO: 2, to nt 52-5340 of SEQ ID NO: 4, to nt 52-5361 of SEQ ID NO: 6, or to nt 220-2898 of SEQ ID NO: 8. Said *CRL* clubroot resistance gene, or *CRL* gene may further comprise an intron, and can comprise a nucleotide sequence having at least 80%, or at least 85%, or at least 90%, or at least 92%, or at least 95%, or at least 98%, or at least 99%, or 100% sequence identity to nt 32750 to 51049 of SEQ ID NO: 1, or can comprise a nucleotide sequence having at least 80%, or at least 85%, or at least 90%, or at least 92%, or at least 95%, or at least 98%, or at least 99%, or 100% sequence identity to nt 32750 to 51049 without transposon region, i.e. to the contiguous sequence of nt 32750-45078 and nt 47953-51049 of SEQ ID NO: 1.

A *CRL*1 clubroot resistance gene, as used herein, is a *CRL* clubroot resistance gene encoding the CRL1 protein having an amino acid sequence having at least 80%, or at least 85%, or at least 90%, or at least 92%, or at least 95%, or at least 98%, or at least 99%, or 100% sequence identity to SEQ ID NO: 3 (CRL1a protein), or to SEQ ID NO: 5 (CRL1b protein), to SEQ ID NO: 7 (CRL1c protein), or to SEQ ID NO: 11 (CRL1d protein). The *CRL*1 clubroot resistance gene may comprise a coding sequence having at least 80%, or at least 85%, or at least 90%, or at least 92%, or at least 95%, or at least 98%, or at least 99%, or 100% sequence identity to nt 52-5343 of SEQ ID NO: 2 (*CRL*1a coding sequence), to nt 52-5340 of SEQ ID NO: 4 (*CRL*1b coding sequence), to nt 52-5361 of SEQ ID NO: 6 (*CRL*1c coding sequence) or to SEQ ID NO: 10 (*CRL*1d d coding sequence). The *CRL*1 clubroot resistance gene may contain an intron, and can comprise a nucleotide sequence having at least 80%, or at least 85%, or at least 90%, or at least 92%, or at least 95%, or at least 98%, or at least 99%, or 100% sequence identity to nt 32750 to 51049 of SEQ ID NO: 1, or to nt 32750 to 50758 of SEQ ID NO: 1, or to nt 32903 to 51049 of SEQ ID NO: 1.

A *CRL2* clubroot resistance gene, as used herein, is a *CRL* clubroot resistance gene encoding the CRL2 protein having an amino acid sequence having at least 80%, or at least 85%, or at least 90%, or at least 92%, or at least 95%, or at least 98%, or at least 99%, or 100% sequence identity to SEQ ID NO: 9. The *CRL2* clubroot resistance gene may comprise a coding sequence having at least 80%, or at least 85%, or at least 90%, or at least 92%, or at least 95%, or at least 98%, or at least 99%, or 100% sequence identity to nt 220-2898 of SEQ ID NO: 8. The *CRL*1 clubroot resistance gene may comprise a nucleotide sequence having at least 80%, or at least 85%, or at least 90%, or at least 92%, or at least 95%, or at least 98%, or at least 99%, or 100% sequence identity to SEQ ID NO: 8.

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, i.e., a position in an alignment where a residue is present in one sequence but not in the other, is regarded as a position with non-identical residues. The "optimal alignment" of two sequences is found by aligning the two sequences over the entire length according to the Needleman and Wunsch global alignment algorithm (Needleman and Wunsch, 1970, J Mol Biol 48(3):443-53) in The European Molecular Biology Open Software Suite (EMBOSS, Rice et al., 2000, Trends in Genetics 16(6): 276-277; see e.g. http://www.ebi.ac.uk/emboss/align/index.html) using default settings (gap opening penalty = 10 (for nucleotides) / 10 (for proteins) and gap extension penalty = 0.5 (for nucleotides) / 0.5 (for proteins)). For nucleotides the default scoring matrix used is EDNAFULL and for proteins the default scoring matrix is EBLOSUM62. It will be clear that whenever nucleotide sequences of RNA molecules are defined by reference to nucleotide sequence of corresponding DNA molecules, the thymine (T) in the nucleotide sequence should be replaced by uracil (U). Whether reference is made to RNA or DNA molecules will be clear from the context of the application.

"Stringent hybridization conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tₘ) for the specific sequences at a defined ionic strength and pH. The Tₘ is the temperature (under defined ionic strength and pH) at which 50% of the target sequence hybridizes to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridizations (Northern blots using a probe of e.g. 100nt) are for example those which include at least one wash in 0.2X SSC at 63°C for 20min, or equivalent conditions.

"High stringency conditions" can be provided, for example, by hybridization at 65°C in an aqueous solution containing 6x SSC (20x SSC contains 3.0 M NaCl, 0.3 M Na-citrate, pH 7.0), 5x Denhardt's (100X Denhardt's contains 2% Ficoll, 2% Polyvinyl pyrollidone, 2% Bovine Serum Albumin), 0.5% sodium dodecyl sulphate (SDS), and 20 µg/ml denaturated carrier DNA (single-stranded fish sperm DNA, with an average length of 120 - 3000 nucleotides) as non-specific competitor. Following hybridization, high stringency washing may be done in several steps, with a final wash (about 30 min) at the hybridization temperature in 0.2-0.1× SSC, 0.1% SDS.

"Moderate stringency conditions" refers to conditions equivalent to hybridization in the above described solution but at about 60-62°C. Moderate stringency washing may be done at the hybridization temperature in 1x SSC, 0.1% SDS.

"Low stringency" refers to conditions equivalent to hybridization in the above described solution at about 50-52°C. Low stringency washing may be done at the hybridization temperature in 2x SSC, 0.1% SDS. See also Sambrook *et al.* (1989) and Sambrook and Russell (2001).

*A "CRL* clubroot resistance locus" as used herein refers to the genetic locus that comprises a *CRL* clubroot resistance gene. A *"CRL* clubroot resistance locus" refers to the position on the chromosome where a *"CRL* clubroot resistance gene" is located. This position can be identified by the location on the genetic map of a chromosome. Included in this definition is the fragment (or segment) of genomic DNA of the chromosome on which the *CRL* clubroot resistance locus is located. Said *CRL* clubroot resistance gene can be a native *CRL* clubroot resistance gene in its native chromosomal position, or can be a transgene on a chromosomal position on which it does not occur naturally. *Brassica* seeds comprising the *CRL* clubroot resistance locus have been deposited at the NCIMB (NCIMB Ltd, Ferguson Building, Craibstone Estate, Bucksburn, Aberdeen AB21 9YA, Scotland, UK) on 7 December 2015, under accession number NCIMB 42499 and on 22 July 2016, under accession number NCIMB 42610.

The *CRL* clubroot resistance locus can comprise the *CRL1* and *CRL2* clubroot resistance genes according to the invention. The *CRL* clubroot resistance locus can comprise a sequence having at least 80%, or at least 85%, or at least 90%, or at least 92%, or at least 95%, or at least 98%, or at least 99%, or 100% sequence identity to SEQ ID NO: 1.

A "locus" as used herein is the position that a gene occupies on a chromosome. A "clubroot resistance locus" refers to the position on the chromosome where a "clubroot resistance gene" is located. This position can be identified by the location on the genetic map of a chromosome. Included in this definition is the fragment (or segment) of genomic DNA of the chromosome on which the clubroot resistance locus is located. Said clubroot resistance gene can be a native clubroot resistance gene in its native chromosomal position, or can be a transgene on a chromosomal position on which it does not occur naturally. Said clubroot resistance gene can be the *CRL* clubroot resistance gene or another clubroot resistance gene. A locus which does not comprise the *CRL* clubroot resistance gene according to the invention, which is at the position on the chromosome corresponding to the position where the *CRL* clubroot resistance gene is located in a resistant line, can be referred to as *"CRL* clubroot susceptibility locus".

As used herein, the term "plant-expressible promoter" means a DNA sequence that is capable of controlling (initiating) transcription in a plant cell. This includes any promoter of plant origin, but also any promoter of non-plant origin which is capable of directing transcription in a plant cell, i.e., certain promoters of viral or bacterial origin such as the CaMV35S (Harpster et al. (1988) Mol Gen Genet. 212(1):182-90, the subterranean clover virus promoter No 4 or No 7 (WO9606932), or T-DNA gene promoters but also tissue-specific or organ-specific promoters including but not limited to seed-specific promoters (e.g., WO89/03887), organ-primordia specific promoters (An et al. (1996) Plant Cell 8(1):15-30), stem-specific promoters (Keller et al., (1988) EMBO J. 7(12): 3625-3633), leaf specific promoters (Hudspeth et al. (1989) Plant Mol Biol. 12: 579-589), mesophyl-specific promoters (such as the light-inducible Rubisco promoters), root-specific promoters (Keller et al. (1989) Genes Dev. 3: 1639-1646), tuber-specific promoters (Keil et al. (1989) EMBO J. 8(5): 1323-1330), vascular tissue specific promoters (Peleman et al. (1989) Gene 84: 359-369), stamen-selective promoters (WO 89/10396, WO 92/13956), dehiscence zone specific promoters (WO 97/13865) and the like.

Suitable promoters for the invention are constitutive plant-expressible promoters. Constitutive plant-expressible promoters are well known in the art, and include the CaMV35S promoter (Harpster et al. (1988) Mol Gen Genet. 212(1):182-90), Actin promoters, such as, for example, the promoter from the Rice Actin gene (McElroy et al., 1990, Plant Cell 2:163), the promoter of the Cassava Vein Mosaic Virus (Verdaguer et al., 1996 Plant Mol. Biol. 31: 1129), the GOS promoter (de Pater et al., 1992, Plant J. 2:837), the Histone H3 promoter (Chaubet et al., 1986, Plant Mol Biol 6:253), the Agrobacterium tumefaciens Nopaline Synthase (Nos) promoter (Depicker et al., 1982, J. Mol. Appl. Genet. 1: 561), or Ubiquitin promoters, such as, for example, the promoter of the maize Ubiquitin-1 gene (Christensen et al., 1992, Plant Mol. Biol. 18:675).

A further promoter suitable for the invention is the endogenous promoter driving expression of the gene encoding an CRL protein.

A "transcription termination and polyadenylation region" as used herein is a sequence that drives the cleavage of the nascent RNA, whereafter a poly(A) tail is added at the resulting RNA 3' end, functional in plant cells. Transcription termination and polyadenylation signals functional in plant cells include, but are not limited to, 3'nos, 3'35S, 3'his and 3'g7.

"Brassicaceae" or "Brassicaceae plant" as used herein refers to plants belonging to the family of Brassicaceae plants, also called Cruciferae or mustard family. Examples of Brassicaceae are, but are not limited to, *Brassica* species, such as *Brassica napus, Brassica oleracea, Brassica rapa, Brassica carinata, Brassica nigra,* and *Brassica juncea; Raphanus* species, such as *Raphanus caudatus, Raphanus raphanistrum,* and *Raphanus sativus; Matthiola* species; *Cheiranthus* species; *Camelina* species, such as *Camelina sativa; Crambe* species, such as *Crambe abyssinica* and *Crambe hispanica; Eruca* species, such as *Eruca vesicaria; Sinapis* species such as *Sinapis alba; Diplotaxis* species; *Lepidium* species; *Nasturtium* species; *Orychophragmus* species; *Armoracia* species, *Eutrema* species; *Lepidium* species; and *Arabidopsis* species.

*A "Brassica* plant" refers to allotetraploid or amphidiploid *Brassica napus* (AACC, 2n=38), *Brassica juncea* (AABB, 2n=36), *Brassica carinata* (BBCC, 2n=34), or to diploid *Brassica rapa* (syn. *B. campestris)* (AA, 2n=20), *Brassica oleracea* (CC, 2n=18) or *Brassica nigra* (BB, 2n=16).

The CRL protein according to the invention may comprise a conserved Coil-Coil domain (CC-domain), EDVID motif, NBS domain, RNBS-A region, RNBS-B region, RNBS-C region, RNBS-D region, a GLPLA (or hydrophobic) region, ARC1 domain, ARC2 domain, LRR domain (leucine rich region) with one or more LRR motifs (xxLxLxx), P-loop kinase1 region (also known as Walker A), Kinase-2 region (with Walker B region), LRRNT region (N-terminal cap, also known as MHD-region) LRRCT region (C-terminal cap) (see, Meyers et al (2003), Plant Cell 15:809; van Ooijen et al (2008) J Exp Bot 59:1383; McHale et al (2006), Genome Biol 7: 212; Bouktila et al (2014) Genet Mol Biol 37: 598 (all documents herein incorporated by reference).

The positions of the conserved domains in the CRL proteins according to the invention in shown in Table 1 and in Figure 1.

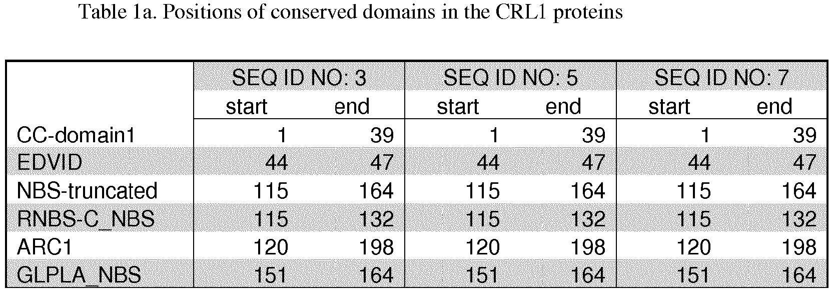

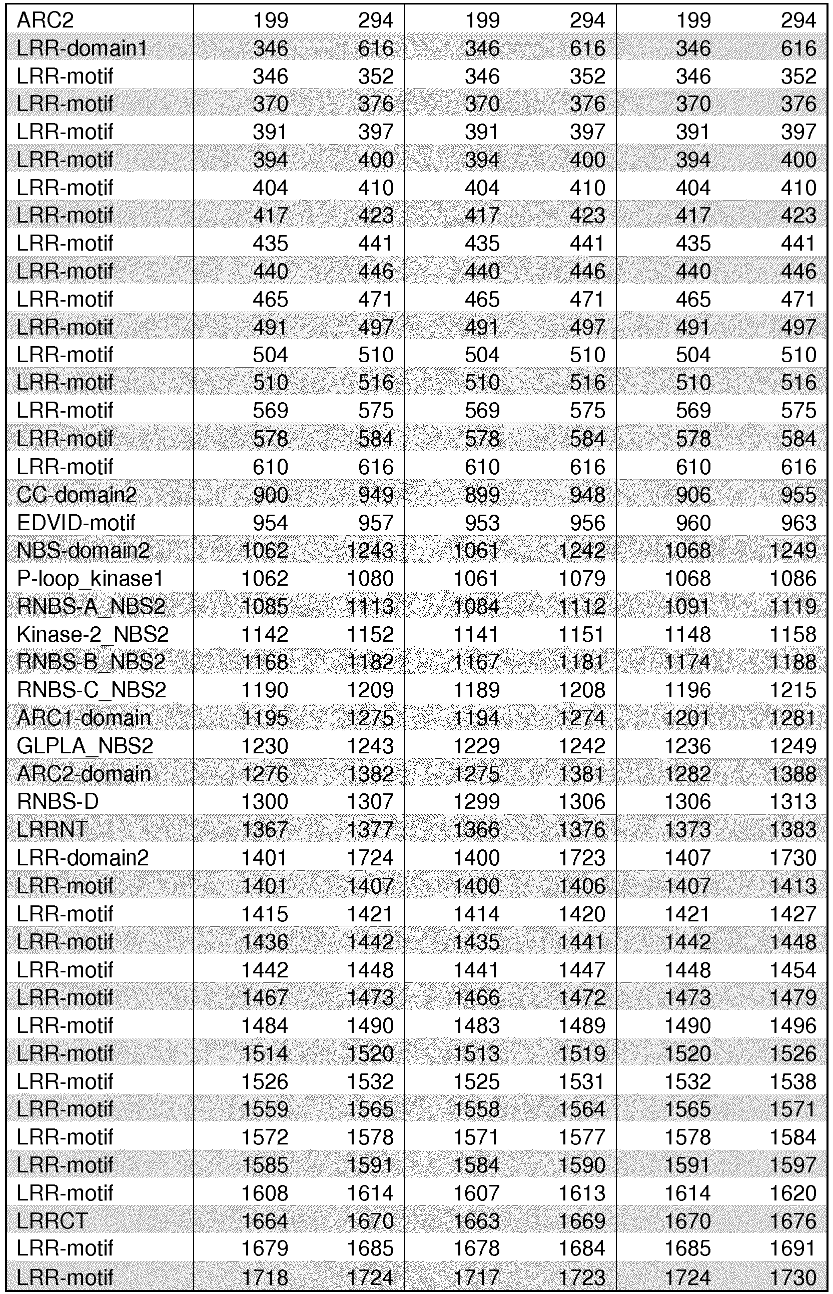

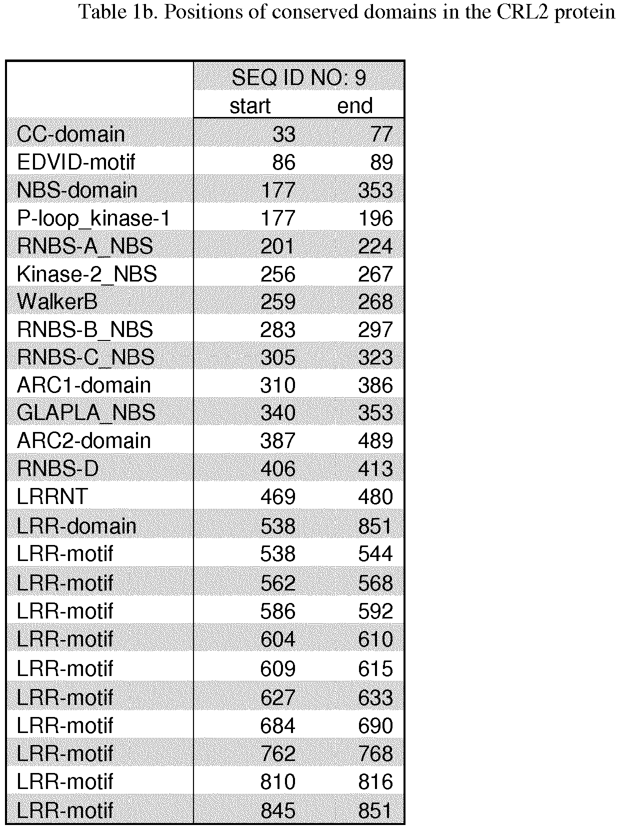

It is another object of the invention to provide a method for increasing clubroot resistance in a Brassicaceae plant, said method comprising introducing or providing a *CRL1* and a *CRL2* clubroot resistance gene according to the invention as a transgene , to a Brassicaceae plant cell, to create transgenic cells; and regenerating transgenic plants from said transgenic cells.

A transgene can be provided to a plant or plant cell using methods well-known in the art. Methods for introduction of genes into plant cells to create transgenic plants are not deemed critical for the current invention and any method to provide plant cells with a transgene suitable for a particular plant species can be used. Such methods are well known in the art and include *Agrobacterium-mediated* transformation, particle gun delivery, microinjection, electroporation of intact cells, polyethyleneglycol-mediated protoplast transformation, electroporation of protoplasts, liposome-mediated transformation, silicon-whiskers mediated transformation etc. Said transgene may be stably integrated into the genome of said plant cell, resulting in a transformed plant cell. The transformed plant cells obtained in this way may then be regenerated into mature fertile transformed plants.

In yet another embodiment, a method is provided for increasing clubroot resistance in a Brassicaceae plant, comprising the step of introducing a *CRL* clubroot resistance locus in said Brassicaceae plant, and selecting said clubroot resistant Brassicaceae plant for the presence of the *CRL* clubroot resistance locus by analyzing genomic DNA from said plant for the presence of at least one molecular marker, wherein said at least one molecular marker is linked to the *CRL* clubroot resistance locus, wherein said *CRL* clubroot resistance locus comprises the *CRL1* and *CRL2* clubroot resistance genes, wherein said *CRL*1 clubroot resistance gene comprises a nucleotide sequence having at least 90% sequence identity to nt 32750 to 51049 of SEQ ID NO: 1 or to nt 32750 to 50758 of SEQ ID NO: 1, or to nt 32903 to 51049 of SEQ ID NO: 1; having a coding sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or encoding a protein having at least 90% sequence identity to SEQ ID NO: 3, SEQ ID NO: 5, SEQ ID NO: 7, or SEQ ID NO: 11; and wherein said *CRL2* clubroot resistance gene comprises a nucleotide sequence having a coding sequence having at least 90% sequence identity to nt 220-2898 of SEQ ID NO: 8; or encoding a protein having at least 90% sequence identity to SEQ ID NO: 9, such as a *CRL* clubroot resistance locus comprising a sequence having at least 90% sequence identity to SEQ ID NO: 1. Said method can comprise the provision of a first Brassicaceae plant comprising a *CRL* clubroot resistance locus, providing a second Brassicaceae plant lacking a *CRL* clubroot resistance locus, crossing the first Brassicaceae plant with the second Brassicaceae plant to provide progeny Brassicaceae plant; analyzing said progeny Brassicaceae plant to determine the presence of a *CRL* clubroot resistance locus by analyzing genomic DNA from the plant for the presence of at least one molecular marker, wherein said at least one molecular marker is linked to the *CRL* clubroot resistance locus; and selecting Brassicaceae progeny that tests positive for the presence of the *CRL* clubroot resistance locus as being Brassicaceae plant into which the *CRL* clubroot resistance locus has been introgressed. Said first Brassicaceae plant may be obtained by screening a population of Brassicaceae plants for the presence of *a CRL* clubroot resistance locus by analyzing genomic DNA from the plant for at least one molecular marker, wherein said at least one molecular marker is linked to the *CRL* clubroot resistance locus. Said first Brassicaceae plant and said progeny Brassicaceae plant may be *Brassica rapa;* and first Brassicaceae plant and said progeny Brassicaceae plant may be *Brassica napus,* or said first Brassicaceae plant may be *Brassica rapa,* said second Brassicaceae plant may be *Brassica oleracea,* and said progeny Brassicaceae plant may be *Brassica napus* obtained through an interspecific cross between said first and said second Brassicaceae plant.

Yet another embodiment provides a method for producing a clubroot resistant Brassicaceae plant comprising the steps of identifying a clubroot resistant Brassicaceae plant comprising a *CRL* clubroot resistance locus according to the invention by analyzing genomic DNA from said plant for the presence of at least one molecular marker, wherein said at least one molecular marker is linked to said *CRL* clubroot resistance locus, and generating progeny from said clubroot resistant Brassicaceae plant, wherein said progeny is clubroot resistant and comprises said *CRL* clubroot resistance locus. In a further embodiment, said *CRL* clubroot resistance locus comprises a sequence having at least 90% sequence identity to SEQ ID NO: 1.

A Brassicaceae plant comprising said *CRL* clubroot resistance locus can be a Brassicaceae plant obtainable from the seed deposited at the NCIMB (NCIMB Ltd, Ferguson Building, Craibstone Estate, Bucksburn, Aberdeen AB21 9YA, Scotland, UK) on 7 December 2015, under accession number NCIMB 42499 or from the seed deposited at the NCIMB (NCIMB Ltd, Ferguson Building, Craibstone Estate, Bucksburn, Aberdeen AB21 9YA, Scotland, UK) on 22 July 2016, under accession number NCIMB 42610.

A "molecular marker", or a "marker", as used herein, refers to a polymorphic locus, i.e. a polymorphic nucleotide (a so-called single nucleotide polymorphism or SNP) or a polymorphic DNA sequence (which can be insertion of deletion of a specific DNA sequence at a specific locus, or polymorphic DNA sequences). A marker refers to a measurable, genetic characteristic with a fixed position in the genome, which is normally inherited in a Mendelian fashion, and which can be used for mapping of a trait of interest. Thus, a molecular marker may be a short DNA sequence, such as a sequence surrounding a single base-pair change, i.e. a single nucleotide polymorphism or SNP, or a long DNA sequence, such as microsatellites or Simple Sequence Repeats (SSRs). The nature of the marker is dependent on the molecular analysis used and can be detected at the DNA, RNA or protein level. Genetic mapping can be performed using molecular markers such as, but not limited to, RFLP (restriction fragment length polymorphisms; Botstein et al. (1980), Am J Hum Genet 32:314-331; Tanksley et al. (1989), Bio/Technology 7:257-263), RAPD [random amplified polymorphic DNA; Williams et al. (1990), NAR 18:6531-6535], AFLP [Amplified Fragment Length Polymorphism; Vos et al. (1995) NAR 23:4407-4414], SSRs or microsatellites [Tautz et al. (1989), NAR 17:6463-6471]. Appropriate primers or probes are dictated by the mapping method used.

The term "marker allele" refers to the version of the marker that is present in a particular plant at one of the chromosomes. Typically, a marker can exist as or can be said to have or to comprise two marker alleles. The term "haplotype", as used herein, refers to a specific combination of marker alleles as present within a certain plant or group of (related) plants. As described herein, a marker allele can be the version of the marker that is present in the resistant line *(CRL* clubroot resistance marker allele). The version of the same marker that is present in the susceptible line can be referred to as *CRL* clubroot susceptibility marker allele.

The term "AFLP^{®}" (AFLP^{®} is a registered trademark of KeyGene N.V., Wageningen, The Netherlands), "AFLP analysis" and "AFLP marker" is used according to standard terminology [Vos et al. (1995), NAR 23:4407-4414; EP0534858; http://www.keygene.com/keygene/techs-apps/]. Briefly, AFLP analysis is a DNA fingerprinting technique which detects multiple DNA restriction fragments by means of PCR amplification. The AFLP technology usually comprises the following steps: (i) the restriction of the DNA with two restriction enzymes, preferably a hexa-cutter and a tetra-cutter, such as EcoRI, PstI and MseI; (ii) the ligation of double-stranded adapters to the ends of the restriction fragments, such as EcoRI, PstI and MseI adaptors; (iii) the amplification of a subset of the restriction fragments using two primers complementary to the adapter and restriction site sequences, and extended at their 3' ends by one to three "selective" nucleotides, i.e., the selective amplification is achieved by the use of primers that extend into the restriction fragments, amplifying only those fragments in which the primer extensions match the nucleotides flanking the restriction sites. AFLP primers thus have a specific sequence and each AFLP primer has a specific code (the primer codes and their sequences can be found at the Keygene website: http://www.keygene.com/keygene/pdf/PRIMERCO.pdf; herein incorporated by reference); (iv) gel electrophoresis of the amplified restriction fragments on denaturing slab gels or cappilaries; (v) the visualization of the DNA fingerprints by means of autoradiography, phosphor-imaging, or other methods. Using this method, sets of restriction fragments may be visualized by PCR without knowledge of nucleotide sequence. An AFLP marker, as used herein, is a DNA fragment of a specific size, which is generated and visualized as a band on a gel by carrying out an AFLP analysis. Each AFLP marker is designated by the primer combination used to amplify it, followed by the approximate size (in base pairs) of the amplified DNA fragment. It is understood that the size of these fragments may vary slightly depending on laboratory conditions and equipment used. Every time reference is made herein to an AFLP marker by referring to a primer combination and the specific size of a fragment, it is to be understood that such size is approximate, and comprises or is intended to include the slight variations observed in different labs. Each AFLP marker represents a certain locus in the genome.

The term "SSR" refers to Simple Sequence Repeats or microsatellite [Tautz et al. (1989), NAR 17:6463-6471]. Short Simple Sequence stretches occur as highly repetitive elements in all eukaryotic genomes. Simple sequence loci usually show extensive length polymorphisms. These simple sequence length polymorphisms (SSLP) can be detected by polymerase chain reaction (PCR) analysis and be used for identity testing, population studies, linkage analysis and genome mapping.

It is understood that molecular markers can be converted into other types of molecular markers. When referring to a specific molecular marker in the present invention, it is understood that the definition encompasses other types of molecular markers used to detect the genetic variation originally identified by the specific molecular markers. For example, if an AFLP marker is converted into another molecular marker using known methods, this other marker is included in the definition. For example, AFLP markers can be converted into sequence-specific markers such as, but not limited to STS (sequenced-tagged-site) or SCAR (sequence-characterized-amplified-region) markers using standard technology as described in Meksem et al. [(2001), Mol Gen Genomics 265(2):207-214], Negi et al. [(2000), TAG 101:146-152], Barret et al. (1989), TAG 97:828-833], Xu et al. [(2001), Genome 44(1):63-70], Dussel et al. [(2002), TAG 105:1190-1195] or Guo et al. [(2003), TAG 103:1011-1017]. For example, Dussel et al. [(2002), TAG 105:1190-1195] converted AFLP markers linked to resistance into PCR-based sequence tagged site markers such as indel (insertion/deletion) markers and CAPS (cleaved amplified polymorphic sequence) markers.

Suitable molecular markers are, for example SNP markers (Single Nucleotide Polymorphisms), AFLP markers, microsatellites, minisatellites, Random Amplified Polymorphic DNA's (RAPD) markers, RFLP markers, Sequence Characterized Amplified Regions (SCAR) markers, and others, such as TRAP markers described by Hu et al. 2007, Genet Resour Crop Evol 54: 1667-1674).

Methods and assays for marker detection, or for analyzing the genomic DNA for the presence of a marker, are widely known in the art. The presence of a marker can, for example be detected in hybridization-based methods (e.g. allele-specific hybridization ), using Taqman, Invader, PCR-based methods, oligonucleotide ligation based methods, or sequencing-based methods.

A useful assay for detection of SNP markers is for example KBioscience Competitive Allele - Specific PCR . For developing the KASP-assay 70 base pairs upstream and 70 basepairs downstream of the SNP are selected and two allele-specific forward primers and one allele specific reverse primer is designed. See e.g. Allen et al. 2011, Plant Biotechnology J. 9, 1086-1099, especially p1097-1098 for KASP assay method (incorporated herein by reference).

Suitable for analyzing the genomic DNA for the presence of a marker are the primers as specified in Table 2. The primers indicated as Primer 1 can be used together with the common primer to detect the marker allele specific for the *CRL* clubroot resistance locus *(CRL* clubroot resistance marker allele). The primers indicated as Primer 2 can be used together with the common primer to detect the marker allele specific for the *CRL* clubroot susceptibility locus *(CRL* clubroot resistance susceptibility allele). The three primers for each marker as specified in Table 2 can be used together in a KASP assay to detect the presence of the *CRL* clubroot resistance locus and of the *CRL* clubroot susceptibility locus.

A "molecular marker linked to the *CRL* clubroot resistance locus", or a "molecular marker linked to the presence of the *CRL* clubroot resistance locus " as used herein refers to a molecular marker in a region in the genome that inherits with the *CRL* clubroot resistance locus as a single genetic unit in at least 50% of the cases. Thus, in this respect, the term linked can be a separation of about 50 cM, or less such as about 40 cM, about 30 cM, about 20 cM, about 10 cM, about 7.5 cM, about 6 cM, about 5 cM, about 4 cM, about 3 cM, about 2.5 cM, about 2 cM, or even less. Particular examples of markers linked to the *CRL* clubroot resistance locus are specified in Table 2. Said "molecular marker linked to the *CRL* clubroot resistance locus" is thus a marker which is linked to the *CRL* clubroot resistance gene. Said marker can be based on the *CRL* clubroot resistance gene itself, such as presence or absence of the *CRL* clubroot resistance gene.

A "molecular marker linked to the *CRL* clubroot resistance locus", or a "molecular marker linked to the presence of the *CRL* clubroot resistance locus " can also be a marker located in a marker interval between and including markers mBRS00013411 and mBRS00013511. Such a marker can thus be any marker at a position on a chromosome between markers mBRS00013411 and mBRS00013511, including markers mBRS00013411 and mBRS00013511. Examples of markers located in a marker interval between and including markers mBRS00013411 and mBRS00013511 are the markers as specified in Table 2.

Suitable are markers that are linked to the *CRL* clubroot resistance locus can be developed using methods known in the art. New markers suitable for the invention can be developed based on the *CRL* sequence. It is understood that such markers can be developed by comparing the sequence of the *CRL* clubroot resistance locus from the resistant Brassicaceae line with the sequence of the same locus in a susceptible Brassicaceae line; identifying a specific sequence region in the *CRL* clubroot resistance locus which does not occur in the corresponding locus of the susceptible Brassicaceae line. A molecular marker linked to the *CRL* clubroot resistance locus can thus be a marker detecting the presence of the *CRL* clubroot resistance locus, or can be a marker directly detecting the presence of the sequence of SEQ ID NO: 1. A molecular marker linked to the *CRL* clubroot resistance locus can also be a marker in the sequences flanking the *CRL* clubroot resistance locus, which is polymorphic between lines comprising the *CRL* clubroot resistance locus and lines lacking, but which inherits with the *CRL* clubroot resistance locus as a single genetic unit in at least 50% of the cases. Suitable primers to detect the presence of the *CRL* clubroot resistance locus are Primer 1 and the common primer of any one of the markers mBRS00013440, mBRS00154152, mBRS00013441, and mBRS00161831.

Markers suitable to determine the presence of the *CRL* clubroot resistance locus can be the markers that are linked to *CRL* clubroot resistance locus, such as the markers the of Table 2, in particular the *CRL* clubroot resistance marker alleles with the polymorphic base detected by Primer 1 and indicated with R.

The absence of the *CRL* clubroot resistance locus can be determined by the absence of marker alleles that are linked to the presence of the *CRL* clubroot resistance locus *(CRL* clubroot resistance marker alleles), such as by the absence of the *CRL* clubroot resistance marker alleles of Table 2 with the polymorphic base detected by Primer 1 and indicated with R. Furthermore, markers suitable to determine the absence of the *CRL* clubroot resistance locus can be marker alleles which are linked to the *CRL* clubroot susceptibility locus *(CRL* clubroot susceptibility marker alleles). Examples of *CRL* clubroot susceptibility marker alleles that are linked to the *CRL* clubroot susceptibility locus are the marker alleles of Table 2 with the polymorphic base detected by Primer 2 and indicated with S.

Analysis for the presence of markers according to the invention can be performed with a first primer and a second primer, and, optionally, a probe, selected from the group consisting of a first primer consisting of a sequence of 15 to 30 nucleotides, or 15 to 25 nucleotides, or 18 to 22 nucleotides of the *CRL* clubroot resistance genes according to the invention, a second primer being complementary to a sequence of 15 to 30 nucleotides, or 15 to 25 nucleotides, or 18 to 22 nucleotides of the *CRL* clubroot resistance genes according to the invention, and wherein the distance between said first and said second primer on the *CRL* clubroot resistance gene is between 1 and 400 bases, or between 1 and 150 bases, and wherein the first primer is located, with respect to the *CRL* coding sequence, upstream of said second primer, and a probe which is identical to at least 15 nucleotides, or at least 18 nucleotides, but not more than 25 nucleotides, or not more than 22 nucleotides of the sequence of the *CRL* clubroot resistance gene between said first and said second primer, provided that either the sequence of the first primer, or the sequence of the second primer, or the sequence of said probe is not present in the corresponding locus in a susceptible Brassicaceae plant. Said probe may be labelled, such as, for example, described in US patent 5,538,848.

Analysis for the presence of markers according to the invention can be performed with a first and second primer as described above recognizing both the *CRL* sequence and the corresponding locus in the susceptible Brassicaceae line, a first probe recognizing a sequence of the *CRL* clubroot resistance gene as described above, but not recognizing a sequence between said first and said second primer in the susceptible Brassicaceaea line, and a second probe recognizing a sequence between said first and said second primer in the susceptible Brassicaceaea line, but not of the *CRL* clubroot resistance gene, and wherein said the label of the first probe is different from that of the second probe.

Further suitable primers for analysis of the presence of markers according to the invention are markers a first primer as described above recognizing both the *CRL* sequence and the corresponding locus in the susceptible Brassicaceae line, a second primer recognizing the *CRL* sequence but not the corresponding locus in the susceptible Brassicaceae line, and a third primer recognizing the corresponding locus in the susceptible Brassicaceae line but not the *CRL* sequence. Said second and third primer may be labelled as indicated above, and said second primer may contain a label which is different from said third primer.

Identification of PCR products specific for the *CRL* clubroot resistance genes and for the corresponding locus in the susceptible Brassicaceae line can occur e.g. by size estimation after gel or capillary electrophoresis (e.g. for the *CRL* clubroot resistance locus and for the corresponding locus in the susceptible Brassicaceae line comprising a number of inserted or deleted nucleotides which results in a size difference between the fragments amplified from the *CRL* clubroot resistance locus and for the corresponding locus in the susceptible Brassicaceae, such that said fragments can be visibly separated on a gel); by evaluating the presence or absence of the two different fragments after gel or capillary electrophoresis, whereby the diagnostic PCR amplification of the *CRL* clubroot resistance locus can, optionally, be performed separately from the diagnostic PCR amplification of the corresponding locus in the susceptible line; by direct sequencing of the amplified fragments; or by fluorescence-based detection methods.

A further embodiment provides Brassicaceae plants or plant cells obtainable by the methods according to the invention, such as *Brassica napus, Brassica juncea, Brassica oleracea, Brassica rapa, Brassica nigra* or *Brassica carinata* plants.

In a further embodiment, a clubroot resistant Brassicaceae plant or plant cell according to the invention is provided, comprising the *CRL1* and *CRL2* clubroot resistance genes according to the invention, and at least one other disease resistance gene, said other disease resistance gene selected from the group consisting of a clubroot resistance gene, a blackleg resistance gene, a Sclerotinia resistance gene, a Verticillium resistance gene, a Fusarium resistance gene, an Aster Yellows resistance gene, an Alternaria resistance gene, and a Grey Stem resistance gene. In a further embodiment, said other disease resistance gene is a transgene which is genetically linked with said *CRL* clubroot resistance genes.

Said clubroot resistance gene may be a *Crr2, Crr4, Crr3, CRk, CRc, CR2a, CR2b, pb-3, pb-4, Pb-Bol, Pb-Bo2, Pb-Bo3, Pb-Bo4, Pb-Bo5a, Pb-Bo5b, Pb-Bo8, Pb-Bo9a, Pb-Bo9b, Pb-Bnl, PbBn-01:60-1, PbBn-01:60-2, PbBn-01:60-3, PbBn-01:60-4, PbBn-01:07-1, PbBn-01:07-2, PbBn-01:07-3, , PbBn-e4x04-1, PbBn-a-1, PbBn-l-1, PbBn-l-2, PbBn-k-1, PbBn-k-2. PbBn-k-3, PbBn-Korp-1, PbBn-Korp-2, PbBn-Korp-3, PbBn-Korp-4, PbBn-Korp-5* as described by Piao et al., 2009, supra, or may be a CRa gene as described by Ueno et al., 2012, supra, a Crr1 gene as described by Hatakeyama et al., 2013, supra and in WO2012/039445, or a *CRb* gene as described by Kato et al., 2013, supra (herein incorporated by reference).

Said Blackleg resistance gene may, for example, be BLMR1 and BLMR2 (WO 2011/044694), LepR3 (Larkan et al., 2013, New Phytol 197:595 and WO 2008/101343), or Lem-08-syl (EP 1547462 and US 2005/0142122). Said Sclerotinia resistance gene may be a sclerotinia resistance gene as described in WO 2005/090578.

Said other disease resistance gene may be present in their native chromosomal position. For example, said other disease resistance genes can be introduced by introgression in the plant according to the invention from the cultivar or -species from which they are derived.

A "clubroot resistance gene" as used herein refers to a DNA sequence which confers, or is associated with, enhanced resistance of a plant, such as a Brassicaceae plant, such as a *Brassica* plant, to *Plasmodiophora brassicae,* compared to a plant lacking the resistance gene(s) or having a non-functional (or inactivated) form of the gene(s).

"Clubroot" as used herein refers to the disease caused by the pathogen *Plasmodiophora brassicae.*

"Clubroot resistance" as used herein refers to resistance to one or more *Plasmodiophora brassicae* isolates, such as, but not limited to, resistance to the *Plasmodiophora brassicae* strain Leduc-ss2 corresponding to pathotype 6 (Xue et al., 2008, Plant Disease 92:456). Said resistance refers to a reduction in damage caused by clubroot infection compared to damage caused on control plants. Damage can be assessed as, for example, formation of club-shaped galls on the roots, occurrence of wilting, stunting, yellowing, premature senescence etc. In particular, a reduction in damage is manifested in a reduced yield loss when plants are grown under disease pressure in the field, compared to control plants. Such reduction in yield loss can, for example, be due to the fact that the infection, reproduction, spread or survival of the pathogen is reduced or prevented in plants with enhanced resistance. Said resistance may also refer to plants that are completely resistant, i.e., plants on which no disease symptoms are found.

Clubroot resistance can be assessed using a scale from zero to three: zero: no clubbing, one: <25% of root system clubbed; two: 25 to 50% of root system clubbed; three: >50% of root system clubbed (Humpherson-Jones, 1989, Tests Agro Cult 10:36). The Disease Index (ID) can be calculated using the following equation: 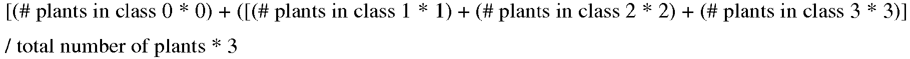 [(# plants in class 0 * 0) + ([(# plants in class 1 * 1) + (# plants in class 2 * 2) + (# plants in class 3 * 3)] / total number of plants * 3 (Strelkov et al., 2006, Can J Plant Pathol 28:467).

It is understood that environmental conditions, such as location, weather conditions and disease pressure, as well as individual perception of the person assessing disease symptoms, can have an effect on the scoring of clubroot resistance. Hence, variation in these factors in comparative tests should be minimized. Any other resistance ratings known in the art can be applied in accordance with this invention to compare the plants of the invention with control plants.

A plant which is clubroot resistant refers to a plant assessed at scale zero or one upon natural infection with the clubroot pathogen, or to a plant assessed at scale zero, one or two upon natural infection with the clubroot pathogen. A clubroot resistant population is a population with a disease index (ID) of less than 30%. A plant with increased clubroot resistance is a plant in which the percentage of the root system which is clubbed is decreased with at least 5%, or at least 10%, or at least 15%, or at least 20%, or at least 30%, or at least 40%, or at least 50%, or at least 70%, or at least 95%, or with 100%, i.e. no clubbing, or refers to a population of plants in which the disease index is reduced with at least 3%, or at least 5%, or at least 8%, or at least 10%, or at least 15%, or at least 20%, or at least 30%, or at least 40%, or at least 50%, or at least 70%, or at least 95%, or with 100%, i.e. all plants of the population are classified in class 0 (no clubbing).

In a further embodiment, said other disease resistance gene is a transgene which is genetically linked with the clubroot resistance gene. A transgene, as used herein, refers to a gene which is stably integrated in the plant cell at another position than where it occurs naturally. A transgene can, for example, be integrated into the genome of a plant cell, or it can be present on an artificial chromosome. A transgene can, for example, be a gene introduced into a plant species or cultivar in which it does not occur naturally, or it can be a gene introduced in a plant species or cultivar in which it does occur naturally, but at another chromosomal position. A transgene may, but does not need to be a chimeric gene. A transgene may, for example, comprise an expression cassette comprising a coding sequence linked to its endogenous promoter. A transgene may also, for example, comprise a coding sequence linked to a heterologous promoter.

The terms "genetically linked", "linked", "linked to" or "linkage", as used herein, refers to a measurable probability that genes or markers located on a given chromosome are being passed on together to individuals in the next generation. Thus, the term "linked" may refer to one or more genes or markers that are passed together with a gene with a probability greater than 0.5 (which is expected from independent assortment where markers/genes are located on different chromosomes). Because the proximity of two genes or markers on a chromosome is directly related to the probability that the genes or markers will be passed together to individuals in the next generation, the term genetically linked may also refer herein to one or more genes or markers that are located within about 50 centimorgan (cM) or less of one another on the same chromosome. Genetic linkage is usually expressed in terms of cM. Centimorgan is a unit of recombinant frequency for measuring genetic linkage, defined as that distance between genes or markers for which one product of meiosis in 100 is recombinant, or in other words, the centimorgan is equal to a 1% chance that a marker at one genetic locus on a chromosome will be separated from a marker at a second locus due to crossing over in a single generation. It is often used to infer distance along a chromosome. The number of base-pairs to which cM correspond varies widely across the genome (different regions of a chromosome have different propensities towards crossover) and the species (i.e. the total size of the genome). Thus, in this respect, the term linked can be a separation of about 50 cM, or less such as about 40 cM, about 30 cM, about 20 cM, about 10 cM, about 7.5 cM, about 6 cM, about 5 cM, about 4 cM, about 3 cM, about 2.5 cM, about 2 cM, or even less. Particular examples of markers linked to the *CRL* clubroot resistance locus are specified in Table 2.

The *CRL* clubroot resistance gene and the other disease resistance gene can be genetically linked when they are stacked as transgenes. For example, *CRL* clubroot resistance gene and the other disease resistance gene can be present on one construct that is used for transformation. Alternatively, the *CRL* clubroot resistance gene can be transformed in a *Brassica* species comprising the other disease resistance gene, provided that it is integrated in the close proximity of the other disease resistance gene, using directed genome engineering techniques. In the latter case, the other disease resistance gene can be present either in its native chromosomal context or as a transgene. Directed genome engineering techniques are, for example, based on homologous recombination, or double-strand break induced targeted integration, or site-specific recombination, such as described in, for example, WO2005/049842, WO2008/148559, WO2011/154158 or WO2011/154159. Alternatively, the other disease resistance gene can be transformed in a *Brassica* species comprising the *CRL* clubroot resistance gene, provided that it is integrated in the close proximity of the *CRL* clubroot resistance gene, using directed genome engineering techniques. In the latter case, the other *CRL* clubroot resistance gene can be present either in its native chromosomal context or as a transgene. The *CRL* clubroot resistance gene and the other disease resistance gene may also be present on an artificial chromosome. "Artificial chromosomes", as used herein are constructs that contain DNA sequences and that perform the critical functions of natural chromosomes that allow them to exist independent (autonomously) from native chromosomes. Autonomy during cell division (mitosis) and gamete formation (meiosis) follows from own functional origins of replication and own functional centromere. Artificial chromosomes are described, for example, in WO 2005/083096 and WO 2007/030510.

In yet another embodiment, the plant according to the invention is selected from the group consisting of *Brassica napus, Brassica juncea, Brassica oleracea, Brassica rapa, Brassica nigra* and *Brassica carinata.* In yet another embodiment, seeds, such as hybrid seeds of the plants according to the invention comprising the *CRL1* and *CRL2* clubroot resistance genes are provided.

Hybrid seeds of the plants according to the invention may be generated by crossing two inbred parental lines, wherein one of the inbred parental lines comprises the *CRL* clubroot resistance genes according to the invention. In order to produce pure hybrid seeds one of the parental lines is male sterile and is pollinated with pollen of the other line. By growing parental lines in rows and only harvesting the F1 seed of the male sterile parent, pure hybrid seeds are produced. To generate male sterile parental lines, the system as described in EP 0,344,029 or US 6,509,516 may be used, wherein a gene encoding a phytotoxic protein (barnase) is expressed under the control of a tapetum specific promoter, such as TA29, ensuring selective destruction of tapetum cells. Transformation of plants with the chimeric gene pTA29:barnase results in plants in which pollen formation is completely prevented [Mariani *et al.*

*(*1990), Nature 347: 737-741]. Cytochemical and histochemical analysis of anther development of *Brassica napus* plants comprising the chimeric pTA29-barnase gene is described by De Block and De Brouwer [(1993), Planta 189:218-225]. To restore fertility in the progeny of a male-sterile plant the male-sterile plant (MS parent) is crossed with a transgenic plant (RF parent) carrying a fertility-restorer gene, which when expressed is capable of inhibiting or preventing the activity of the male-sterility gene [U.S. Pat. Nos. 5,689,041; 5,792,929; De Block and De Brouwer, supra]. The use of co-regulating genes in the production of male-sterile plants to increase the frequency of transformants having good agronomical performance is described in WO96/26283. Typically, when the sterility DNA encodes a barnase, the co-regulating DNA will encode a barstar, preferably an optimized barstar gene is used as described in published PCT patent application WO 98/10081. It is understood that different promoters may be used to drive barnase expression in order to render the plant male sterile. Likewise, barstar may be operably linked to different promoters, such as 35S from Cauliflower mosaic virus.

Male sterile plants can also be generated using other techniques, such as cytoplasmic male sterility/restorer systems [e.g. the Ogura system, published US patent application 20020032916, US 6,229,072, WO97/02737, US 5,789,566 or the Polima system of US 6,365,798, WO98/54340 or the Kosena system of WO95/09910, US 5,644,066].

Either the MS parent or the RF parent, or both, may comprise the *CRL* clubroot resistance genes according to the invention. This can be accomplished by either introducing the *CRL* clubroot resistance genes into an elite *B. napus* line and then transforming this line with pTA29-barnase or with pNOS-barstar using known methods. Alternatively the *CRL* clubroot resistance genes can be introduced directly into a transgenic MS or RF parent line, by crossing a plant comprising the *CRL* clubroot resistance genes with the MS parent or RF-parent, or by transformation of the MS parent or the RF parent. The F1 hybrid seeds generated from the cross between the MS and RF parent will then contain the *CRL* clubroot resistance genes.

A further embodiment provides methods to determine the presence or absence of a *CRL* clubroot resistance locus in a biological sample, comprising providing genomic DNA from said biological sample, and analyzing said DNA for the presence of at least one molecular marker, wherein the at least one molecular marker is linked to the presence or absence of the *CRL* clubroot resistance locus, wherein said *CRL* clubroot resistance locus comprises the *CRL1* and *CRL2* clubroot resistance genes, wherein said *CRL1* clubroot resistance gene comprises a nucleotide sequence having at least 90% sequence identity to nt 32750-51049 of SEQ ID NO: 1 or to nt 32750 to 50758 of SEQ ID NO: 1, or to nt 32903 to 51049 of SEQ ID NO: 1; having a coding sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or encoding a protein having at least 90% sequence identity to SEQ ID NO: 3, SEQ ID NO: 5, SEQ ID NO: 7, or SEQ ID NO: 9; and wherein said *CRL2* clubroot resistance gene comprises a nucleotide sequence having a coding sequence having at least 90% sequence identity to nt 220-2898 of SEQ ID NO: 8; or encoding a protein having at least 90% sequence identity to SEQ ID NO: 9. In yet a further embodiment, a kit is provided for the detection of the *CRL* clubroot resistance locus according to the invention in Brassicaceae DNA samples, wherein said kit comprises at least one primer or probe which specifically recognizes a molecular marker linked to said *CRL* clubroot resistance locus. Yet another embodiment provides the use of a molecular marker linked to the *CRL* clubroot resistance locus according to the invention for determining the presence or absence of said *CRL* clubroot resistance locus in Brassicaceae plants, or the use of the sequence of any one of SEQ ID NOs: 1, 2, 4, 6 and 8 for determining the presence or absence of the *CRL* clubroot resistance locus according to the invention in Brassicaceae plants.

In particular, the methods and kits according to the invention are suitable to determine the presence of the *CRL* clubroot resistance locus. The presence of the *CRL* clubroot resistance locus can be determined using at least one molecular marker, wherein said one molecular marker is linked to the presence of the *CRL* clubroot resistance locus as defined herein.

A "biological sample" can be a plant or part of a plant such as a plant tissue or a plant cell.

"Providing genomic DNA" as used herein refers to providing a sample comprising genomic DNA from the plant. The sample can refer to a tissue sample which has been obtained from said plant, such as, for example, a leaf sample, comprising genomic DNA from said plant. The sample can further refer to genomic DNA which is obtained from a tissue sample, such as genomic DNA which has been obtained from a tissue, such as a leaf sample. Providing genomic DNA can include, but does not need to include, purification of genomic DNA from the tissue sample. Providing genomic DNA thus also includes obtaining tissue material from a plant or larger piece of tissue and preparing a crude extract or lysate therefrom.

A "kit", as used herein, refers to a set of reagents for the purpose of performing the method of the invention, more particularly, the identification of the *CRL* clubroot resistance genes in biological samples or the determination of the zygosity status of plant material comprising the *CRL* clubroot resistance genes. More particularly, a preferred embodiment of the kit of the invention comprises at least two specific primers for identification of the *CRL* clubroot resistance genes, or at least two or three specific primers for the determination of the zygosity status. Optionally, the kit can further comprise any other reagent. Alternatively, according to another embodiment of this invention, the kit can comprise at least one specific probe, which specifically hybridizes with nucleic acid of biological samples to identify the presence of the *CRL* clubroot resistance genes therein, or at least two or three specific probes for the determination of the zygosity status. Optionally, the kit can further comprise any other reagent (such as but not limited to hybridizing buffer, label) for identification of the *CRL* clubroot resistance genes in biological samples, using the specific probe.

The kit of the invention can be used, and its components can be specifically adjusted, for purposes of quality control (e.g., purity of seed lots), detection of the presence or absence of the *CRL* clubroot resistance genes in plant material or material comprising or derived from plant material, such as but not limited to food or feed products. The zygosity status of the *CRL* clubroot resistance genes can be determined by using alternative sets of primers and/or probes that specifically the *CRL* locus and the corresponding locus in a susceptible Brassicaceae line.

The term "primer" as used herein encompasses any nucleic acid that is capable of priming the synthesis of a nascent nucleic acid in a template-dependent process, such as PCR. Typically, primers are oligonucleotides from 10 to 30 nucleotides, but longer sequences can be employed. Primers may be provided in double-stranded form, though the single-stranded form is preferred. Probes can be used as primers, but are designed to bind to the target DNA or RNA and need not be used in an amplification process.

The term "recognizing" as used herein when referring to specific primers, refers to the fact that the specific primers specifically hybridize to a specific nucleic acid sequence under the conditions set forth in the method (such as the conditions of the PCR identification protocol), whereby the specificity is determined by the presence of positive and negative controls.

It is another object of the invention to provide a chimeric gene comprising the following genetic elements: a plant-expressible promoter, a DNA sequence coding for a CRL1 or for a CRL2 protein, and optionally, a transcription termination and polyadenylation region functional in plant cells, wherein said DNA sequence coding for a CRL1 protein comprises a nucleotide sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or encoding a protein having an amino acid sequence having at least 90% sequence identity to ID NO: 3, SEQ ID NO: 5, SEQ ID NO: 7, or SEQ ID NO: 11; and wherein said DNA sequence coding for a of CRL2 protein comprises a nucleotide sequence having at least 90% sequence identity to 220-2898 of SEQ ID NO: 8; or encoding a protein having an amino acid sequence having at least 90% sequence identity to SEQ ID NO: 9.

The chimeric gene comprising the sequence coding for a CRL1 protein can be on the same vector as the chimeric gene comprising the sequence coding for a CRL2 protein which can be used for plant transformation. Alternatively, the chimeric gene comprising the sequence coding for a CRL1 protein and the chimeric gene comprising the sequence coding for a CRL2 protein can be on different vectors which can together be used for plant transformation. Plants can either be transformed with the two vectors simultaneously. Alternatively, plants can first be transformed with one of the vectors to create transgenic plants, and said transgenic plants can subsequently be transformed with the other vector.

As used herein a "chimeric gene" refers to a nucleic acid construct which is not normally found in a plant species. A chimeric nucleic acid construct can be DNA or RNA. "Chimeric DNA construct" and "chimeric gene" are used interchangeably to denote a gene in which the promoter or one or more other regulatory regions of the gene are not associated in nature with part or all of the transcribed DNA region, or a gene which is present in a locus in the plant genome in which it does not occur naturally.

"Isolated DNA" as used herein refers to DNA not occurring in its natural genomic context, irrespective of its length and sequence. Isolated DNA can, for example, refer to DNA which is physically separated from the genomic context, such as a fragment of genomic DNA. Isolated DNA can also be an artificially produced DNA, such as a chemically synthesized DNA, or such as DNA produced via amplification reactions, such as polymerase chain reaction (PCR) well-known in the art. Isolated DNA can further refer to DNA present in a context of DNA in which it does not occur naturally. For example, isolated DNA can refer to a piece of DNA present in a plasmid. Further, the isolated DNA can refer to a piece of DNA present in another chromosomal context than the context in which it occurs naturally, such as for example at another position in the genome than the natural position, in the genome of another species than the species in which it occurs naturally, or in an artificial chromosome.

Further, expression of the *CRL* clubroot resistance gene can be modulated, such as increased by, for example, T-DNA activation tagging, or by targeted genome engineering technologies in which, for example, the endogenous promoter is modified such that it drives higher levels of expression, or in which the endogenous promoter is replaced with a stronger promoter.

Suitable to the invention is a method to produce clubroot free Brassicaceae plants, comprising the steps of sowing seeds from the Brassicaceae plants according to the invention comprising a *CRL* clubroot resistance gene, growing the plants in the field, optionally spraying the plants with fungicides, and harvesting.

A further object provides the use of the chimeric gene according to the invention to increase clubroot resistance in Brassicaceae, and the use of the plants according to the invention to produce oilseed rape oil or an oilseed rape seed cake, or a seed, or a crop of oilseed rape.

The sequence of the *CRL* clubroot resistance locus can further be used to develop molecular markers linked to the *CRL* clubroot resistance locus.

The isolated DNA according to the invention can be used to develop molecular markers for the *CRL* clubroot resistance locus by developing primers specifically recognizing the *CRL* clubroot resistance gene. Further, the isolated DNA can be used to identify the genomic sequence flanking the *CRL* clubroot resistance gene, and develop primers and probes based on the genomic sequences flanking the *CRL* clubroot resistance gene.

Also provided is a method of producing food, feed, or an industrial product, comprising obtaining the plant according to the invention or a part thereof; and preparing the food, feed or industrial product from the plant or part thereof. In a further object, said food or feed is oil, meal, grain, starch, flour or protein; or said industrial product is biofuel, fiber, industrial chemicals, a pharmaceutical or a nutraceutical.

Further provided is the use of the nucleotide sequence of SEQ ID NO: 1, SEQ ID NO: 2, SEQ ID NO: 4, SEQ ID NO: 6, SEQ ID NO: 8,, or SEQ ID NO: 10 or of the amino acid sequence of SEQ ID NO: 3, or SEQ ID NO: 5, or SEQ ID NO: 7, SEQ ID NO: 9, or SEQ ID NO: 11 to identify homologous clubroot resistance genes.

Homologous clubroot resistance genes can be identified using methods known in the art. Homologous nucleotide sequence may be identified and isolated by hybridization under stringent conditions using as probes a nucleic acid comprising the nucleotide sequence of SEQ ID NO: 1, SEQ ID NO: 2, SEQ ID NO: 4, SEQ ID NO: 6, SEQ ID NO: 8, or SEQ ID NO: 10 or part thereof. Other sequences encoding CRL may also be obtained by DNA amplification using oligonucleotides specific for genes encoding CRL as primers, such as but not limited to oligonucleotides comprising or consisting of about 20 to about 50 consecutive nucleotides from SEQ SEQ ID NO: 1, SEQ ID NO: 2, SEQ ID NO: 4, SEQ ID NO: 6, SEQ ID NO: 8, or SEQ ID NO: 10 or its complement. Homologous clubroot resistance genes can be identified *in silico* using Basic Local Alignment Search Tool (BLAST) homology search with other nucleotide or amino acid sequences. Functionality of the identified homologous clubroot resistance genes can be validated using the methods described herein, such as transforming a the clubroot resistance gene under control of a plant-expressible promoter in a plant not being clubroot resistant.

Also provided is a method of producing food, feed, or an industrial product, comprising obtaining the plant according to the invention or a part thereof; and preparing the food, feed or industrial product from the plant or part thereof. In a further object, said food or feed is oil, meal, grain, starch, flour or protein; or said industrial product is biofuel, fiber, industrial chemicals, a pharmaceutical or a nutraceutical.

"Crop of oilseed rape" as used herein refers to oilseed rape cultivated as a crop, such as *Brassica napus, Brassica juncea, Brassica carinata, Brassica rapa* (syn. *B. campestris), Brassica oleracea* or *Brassica nigra.*

The plants according to the invention may additionally contain an endogenous or a transgene, which confers herbicide resistance, such as the bar or pat gene, which confer resistance to glufosinate ammonium (Liberty^{®}, Basta^{®} or Ignite^{®}) [EP 0 242 236 and EP 0 242 246 incorporated by reference]; or any modified EPSPS gene, such as the 2mEPSPS gene from maize [EP0 508 909 and EP 0 507 698 incorporated by reference], or glyphosate acetyltransferase, or glyphosate oxidoreductase, which confer resistance to glyphosate (RoundupReady^{®}), or bromoxynitril nitrilase to confer bromoxynitril tolerance, or any modified AHAS gene, which confers tolerance to sulfonylureas, imidazolinones, sulfonylaminocarbonyltriazolinones, triazolopyrimidines or pyrimidyl(oxy/thio)benzoates, such as oilseed rape imidazolinone-tolerant mutants PM1 and PM2, currently marketed as Clearfield^{®} canola. Further, the plants according to the invention may additionally contain an endogenous or a transgene which confers increased oil content or improved oil composition, such as a 12:0 ACP thioesteraseincrease to obtain high laureate, which confers pollination control, such as such as barnase under control of an anther-specific promoter to obtain male sterility, or barstar under control of an anther-specific promoter to confer restoration of male sterility, or such as the Ogura cytoplasmic male sterility and nuclear restorer of fertility.

The plants and seeds according to the invention may be further treated with a chemical compound, such as a chemical compound selected from the following lists: Herbicides: Clethodim, Clopyralid, Diclofop, Ethametsulfuron, Fluazifop, Glufosinate, Glyphosate, Metazachlor, Quinmerac, Quizalofop, Tepraloxydim, Trifluralin. Fungicides / PGRs: Azoxystrobin, N-[9-(dichloromethylene)-1,2,3,4-tetrahydro-1,4-methanonaphthalen-5-yl]-3-(difluoromethyl)-1-methyl-1H-pyrazole-4-carboxamide (Benzovindiflupyr, Benzodiflupyr), Bixafen, Boscalid, Carbendazim, Carboxin, Chlormequat-chloride, Coniothryrium minitans, Cyproconazole, Cyprodinil, Difenoconazole, Dimethomorph, Dimoxystrobin, Epoxiconazole, Famoxadone, Fluazinam, Fludioxonil, Fluopicolide, Fluopyram, Fluoxastrobin, Fluquinconazole, Flusilazole, Fluthianil, Flutriafol, Fluxapyroxad, Iprodione, Isopyrazam, Mefenoxam, Mepiquat- chloride, Metalaxyl, Metconazole, Metominostrobin, Paclobutrazole, Penflufen, Penthiopyrad, Picoxystrobin, Prochloraz, Prothioconazole, Pyraclostrobin, Sedaxane, Tebuconazole, Tetraconazole, Thiophanate-methyl, Thiram, Triadimenol, Trifloxystrobin, Bacillus firmus, Bacillus firmus strain I-1582, Bacillus subtilis, Bacillus subtilis strain GB03, Bacillus subtilis strain QST 713, Bacillus pumulis, Bacillus. pumulis strain GB34. Insecticides: Acetamiprid, Aldicarb, Azadirachtin, Carbofuran, Chlorantraniliprole (Rynaxypyr), Clothianidin, Cyantraniliprole (Cyazypyr), (beta-)Cyfluthrin, gamma-Cyhalothrin, lambda-Cyhalothrin, Cypermethrin, Deltamethrin, Dimethoate, Dinetofuran, Ethiprole, Flonicamid, Flubendiamide, Fluensulfone, Fluopyram,Flupyradifurone, tau-Fluvalinate, Imicyafos, Imidacloprid, Metaflumizone, Methiocarb, Pymetrozine, Pyrifluquinazon, Spinetoram, Spinosad, Spirotetramate, Sulfoxaflor, Thiacloprid, Thiamethoxam, 1-(3-chloropyridin-2-yl)-N-[4-cyano-2-methyl-6-(methylcarbamoyl)phenyl]-3-{[5-(trifluoromethyl)-2H-tetrazol-2-yl]methyl}-1H-pyrazole-5-carboxamide, 1-(3-chloropyridin-2-yl)-N-[4-cyano-2-methyl-6-(methylcarbamoyl)phenyl]-3-{[5-(trifluoromethyl)-1H-tetrazol-1-yl methyl }-1H-pyrazole-5-carboxamide, 1-{2-fluoro-4-methyl-5-[(2,2,2-trifluorethyl)sulfinyl]phenyl}-3-(trifluoromethyl)-1H-1,2,4-triazol-5-amine, (1E)-N-[(6-chloropyridin-3-yl)methyl]-N'-cyano-N-(2,2-difluoroethyl)ethanimidamide, Bacillus firmus, Bacillus firmus strain I-1582, Bacillus subtilis, Bacillus subtilis strain GB03, Bacillus subtilis strain QST 713, Metarhizium anisopliae F52.

Whenever reference to a "plant" or "plants" according to the invention is made, it is understood that also plant parts (cells, tissues or organs, seed pods, seeds, severed parts such as roots, leaves, flowers, pollen, etc.), progeny of the plants which retain the distinguishing characteristics of the parents (especially the fruit dehiscence properties), such as seed obtained by selfing or crossing, e.g. hybrid seed (obtained by crossing two inbred parental lines), hybrid plants and plant parts derived there from are encompassed herein, unless otherwise indicated.

In some embodiments, the plant cells of the invention, i.e. a plant cell comprising a *CRL* clubroot resistance gene as well as plant cells generated according to the methods of the invention, may be non-propagating cells.

The obtained plants according to the invention can be used in a conventional breeding scheme to produce more plants with the same characteristics or to introduce the characteristic of the presence of the *CRL* gene according to the invention in other varieties of the same or related plant species, or in hybrid plants. The obtained plants can further be used for creating propagating material. Plants according to the invention can further be used to produce gametes, seeds (including crushed seeds and seed cakes), seed oil, embryos, either zygotic or somatic, progeny or hybrids of plants obtained by methods of the invention. Seeds obtained from the plants according to the invention are also encompassed by the invention.

"Creating propagating material", as used herein, relates to any means know in the art to produce further plants, plant parts or seeds and includes inter alia vegetative reproduction methods (e.g. air or ground layering, division, (bud) grafting, micropropagation, stolons or runners, storage organs such as bulbs, corms, tubers and rhizomes, striking or cutting, twin-scaling), sexual reproduction (crossing with another plant) and asexual reproduction (e.g. apomixis, somatic hybridization).

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a nucleic acid which is functionally or structurally defined, may comprise additional DNA regions etc.

The sequence listing contained in the file named "BCS15-2016_ST25.txt", which is 289 kilobytes (size as measured in Microsoft Windows^{®}), contains 176 sequences SEQ ID NO: 1 through SEQ ID NO: 176 is filed herewith by electronic submission and is incorporated by reference herein.

In the description and examples, reference is made to the following sequences:

### SEQUENCES

SEQ ID NO: *1: CRL* genome sequence
SEQ ID NO: 2: *CRL*1a coding sequence
SEQ ID NO: 3: CRL1a protein sequence
SEQ ID NO: 4: *CRL1b* coding sequence
SEQ ID NO: 5: CRL1b protein sequence
SEQ ID NO: 6: *CRL*1c coding sequence
SEQ ID NO: 7: CRL1c protein sequence
SEQ ID NO: 8: *CRL2* coding sequence
SEQ ID NO: 9: CRL2 protein sequence
SEQ ID NO: 10: *CRL*1d coding sequence
SEQ ID NO: 11: CRL1d protein sequence
SEQ ID NO: 12: Primer 12
SEQ ID NO: 13: Primer 13
SEQ ID NO: 14: Primer 14
SEQ ID NO: 15: Primer 15
SEQ ID NO: 16: Primer 16
SEQ ID NO: 17: Primer 17
SEQ ID NO: 18: Primer 18
SEQ ID NO: 19: Primer 19
SEQ ID NO: 20: Primer 20
SEQ ID NO: 21: Primer 21
SEQ ID NO: 22: Primer 22
SEQ ID NO: 23: Primer 23
SEQ ID NO: 24: Primer 24
SEQ ID NO: 25: Primer 25
SEQ ID NO: 26: Primer 26
SEQ ID NO: 27: Primer 27
SEQ ID NO: 28: Primer 28
SEQ ID NO: 29: Primer 29
SEQ ID NO: 30: Primer 30
SEQ ID NO: 31: Primer 31
SEQ ID NO: 32: Primer 32
SEQ ID NO: 33: Primer 33
SEQ ID NO: 34: Primer 34
SEQ ID NO: 35: Primer 35
SEQ ID NO: 36: Primer 36
SEQ ID NO: 37: Primer 37
SEQ ID NO: 38: Primer 38
SEQ ID NO: 39: Primer 39
SEQ ID NO: 40: Primer 40
SEQ ID NO: 41: Primer 41
SEQ ID NO: 42: Primer 42
SEQ ID NO: 43: Primer 43
SEQ ID NO: 44: Primer 44
SEQ ID NO: 45: Primer 45
SEQ ID NO: 46: Primer 46
SEQ ID NO: 47: Primer 47
SEQ ID NO: 48: Primer 48
SEQ ID NO: 49: Primer 49
SEQ ID NO: 50: Primer 50
SEQ ID NO: 51: Primer 51
SEQ ID NO: 52: Primer 52
SEQ ID NO: 53: Primer 53
SEQ ID NO: 54: Primer 54
SEQ ID NO: 55: Primer 55
SEQ ID NO: 56: Primer 56
SEQ ID NO: 57: Primer 57
SEQ ID NO: 58: Primer 58
SEQ ID NO: 59: Primer 59
SEQ ID NO: 60: Primer 60
SEQ ID NO: 61: Primer 61
SEQ ID NO: 62: Primer 62
SEQ ID NO: 63: Primer 63
SEQ ID NO: 64: Primer 64
SEQ ID NO: 65: Primer 65
SEQ ID NO: 66: Primer 66
SEQ ID NO: 67: Primer 67
SEQ ID NO: 68: Primer 68
SEQ ID NO: 69: Primer 69
SEQ ID NO: 70: Primer 70
SEQ ID NO: 71: Primer 71
SEQ ID NO: 72: Primer 72
SEQ ID NO: 73: Primer 73
SEQ ID NO: 74: Primer 74
SEQ ID NO: 75: Primer 75
SEQ ID NO: 76: Primer 76
SEQ ID NO: 77: Primer 77
SEQ ID NO: 78: Primer 78
SEQ ID NO: 79: Primer 79
SEQ ID NO: 80: Primer 80
SEQ ID NO: 81: Primer 81
SEQ ID NO: 82: Primer 82
SEQ ID NO: 83: Primer 83
SEQ ID NO: 84: Primer 84
SEQ ID NO: 85: Primer 85
SEQ ID NO: 86: Primer 86
SEQ ID NO: 87: Primer 87
SEQ ID NO: 88: Primer 88
SEQ ID NO: 89: Primer 89
SEQ ID NO: 90: Primer 90
SEQ ID NO: 91: Primer 91
SEQ ID NO: 92: Primer 92
SEQ ID NO: 93: Primer 93
SEQ ID NO: 94: Primer 94
SEQ ID NO: 95: Primer 95
SEQ ID NO: 96: Primer 96
SEQ ID NO: 97: Primer 97
SEQ ID NO: 98: Primer 98
SEQ ID NO: 99: Primer 99
SEQ ID NO: 100: Primer 100
SEQ ID NO: 101: Primer 101
SEQ ID NO: 102: Primer 102
SEQ ID NO: 103: Primer 103
SEQ ID NO: 104: Primer 104
SEQ ID NO: 105: Primer 105
SEQ ID NO: 106: Primer 106
SEQ ID NO: 107: Primer 107
SEQ ID NO: 108: Primer 108
SEQ ID NO: 109: Primer 109
SEQ ID NO: 110: Primer 110
SEQ ID NO: 111: Primer 111
SEQ ID NO: 112: Primer 112
SEQ ID NO: 113: Primer 113
SEQ ID NO: 114: Primer 114
SEQ ID NO: 115: Primer 115
SEQ ID NO: 116: Primer 116
SEQ ID NO: 117: Primer 117
SEQ ID NO: 118: Primer 118
SEQ ID NO: 119: Primer 119
SEQ ID NO: 120: Primer 120
SEQ ID NO: 121: Primer 121
SEQ ID NO: 122: Primer 122
SEQ ID NO: 123: Primer 123
SEQ ID NO: 124: Primer 124
SEQ ID NO: 125: Primer 125
SEQ ID NO: 126: Primer 126
SEQ ID NO: 127: Primer 127
SEQ ID NO: 128: Primer 128
SEQ ID NO: 129: Primer 129
SEQ ID NO: 130: Primer 130
SEQ ID NO: 131: Primer 131
SEQ ID NO: 132: Primer 132
SEQ ID NO: 133: Primer 133
SEQ ID NO: 134: Primer 134
SEQ ID NO: 135: Primer 135
SEQ ID NO: 136: Primer 136
SEQ ID NO: 137: Primer 137
SEQ ID NO: 138: Primer 138
SEQ ID NO: 139: Primer 139
SEQ ID NO: 140: Primer 140
SEQ ID NO: 141: Primer 141
SEQ ID NO: 142: Primer 142
SEQ ID NO: 143: Primer 143
SEQ ID NO: 144: Primer 144
SEQ ID NO: 145: Primer 145
SEQ ID NO: 146: Primer 146
SEQ ID NO: 147: Primer 147
SEQ ID NO: 148: Primer 148
SEQ ID NO: 149: Primer 149
SEQ ID NO: 150: Primer 150
SEQ ID NO: 151: Primer 151
SEQ ID NO: 152: Primer 152
SEQ ID NO: 153: Primer 153
SEQ ID NO: 154: Primer 154
SEQ ID NO: 155: Primer 155
SEQ ID NO: 156: Primer 156
SEQ ID NO: 157: Primer 157
SEQ ID NO: 158: Primer 158
SEQ ID NO: 159: Primer 159
SEQ ID NO: 160: Primer 160
SEQ ID NO: 161: Primer 161
SEQ ID NO: 162: Primer 162
SEQ ID NO: 163: Primer 163
SEQ ID NO: 164: Primer 164
SEQ ID NO: 165: Primer 165
SEQ ID NO: 166: Primer 166
SEQ ID NO: 167: Primer 167
SEQ ID NO: 168: Primer 168
SEQ ID NO: 169: Primer 169
SEQ ID NO: 170: Primer 170
SEQ ID NO: 171: Primer 171
SEQ ID NO: 172: Primer 172
SEQ ID NO: 173: Primer 173
SEQ ID NO: 174: Primer 174
SEQ ID NO: 175: Primer 175
SEQ ID NO: 176: Primer 176

### EXAMPLES

Unless stated otherwise in the Examples, all recombinant DNA techniques are carried out according to standard protocols as described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and in Volumes I and II of Brown (1998) Molecular Biology LabFax, Second Edition, Academic Press (UK). Standard materials and methods for plant molecular work are described in Plant Molecular Biology Labfax (1993) by R.D.D. Croy, jointly published by BIOS Scientific Publications Ltd (UK) and Blackwell Scientific Publications, UK. Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

### Example 1 - Rough mapping of Clubroot resistance by GGGT analysis

### Generation of mapping population

A DH population was made by microspore regeneration from the F1 cross between a resistant female parent and a susceptible male parent. The population consisted of 106 DH individuals.

### Golden Gate Genotyping (iSCAN 1536-GGGT) analysis

### DNA extraction.

DNA was extracted from 106 DH plants and the 2 corresponding parents (resistant female parent and susceptible male parent) using the automated KingFisher DNA extraction method (Thermo Scientific) using the manufacturer's purification kits and protocol.

### GGGT analysis.

A custom Illumina GoldenGate BeadArray was designed by evaluating individual loci with the Illumina Assay Design Tool (ADT) and selecting most successful custom genotyping assays (score >0.6 (Hyten et al., 2008, Theor Appl Genet 116:945)). A custom 1536 single nucleotide polymorphism (SNP) Oligonucleotide Pool Assay was selected.

Assays were performed as described by the manufacturer's protocol and as described in Fan et al., 2003, Cold Spring Harb Symp Quant Biol 68:69. A total of 250 ng (50 ng/µl) of genomic DNA was used to make single-use DNA. The single-use DNA underwent an allele specific oligonucleotide hybridization, which involves three oligos at each of the 1536 different SNP loci. At each SNP locus, two of the oligos are allele-specific oligos that are complementary to the genomic sequence directly adjacent to the SNP being assayed except they differ at the 3' base in order to be complementary with one of the two SNP alleles and each oligo has a universal primer site attached at the 5' end with one allele for each SNP having universal primer site. Amplification over the oligo region involves three primers, the two allele specific primers having either a Cy3- or Cy5-label allowing allele specific detection after excitation by lasers emitting at 532 nm and 635 nm. A genotype that is homozygous for one SNP allele will display a signal in either the Cy3 or the Cy5 channel, whereas a genotype that is heterozygous will display a signal in both channels. The Locus Specific oligo is labelled with biotin and contains the "IllumniCode" sequence which is unique to each SNP locus. It is the IllumniCode that binds to its complementary sequence attached to a bead on the sentrix array matrix (SAM).

The hybridised SAM was analysed on the Illumina iSCAN system (array reader) using the iScan Control Software (ICS). After images are scanned, they are registered according to the corresponding bead map (*.dmap) file and two-colour signal intensity values are extracted for every bead on the image (*.idat). By default, the ICS AutoConvert was enabled, normalising the intensity data and generating genotype calls.

Analysis of BeadArray idat files was performed using GenomeStudioTM Data Analysis Software (GenomeStudio Software V2011.1 (Illumina)). Primary data analyses, such as raw data normalization, clustering, and genotype calling are performed using integrated algorithms (GenTrain & GenCall) in the GenoTyping (GT) Module. Genotype calls are made from BeadChip marker two-colour signal intensities by comparison to canonical genotype clusters. Cluster position identification is performed by the GenTrain algorithm. With one sample precluded due to failed DNA extraction no further poorly performing samples were identified so further sample removal and reclustering of all SNPs was not required. Never-the-less the position of SNP clusters required editing by visualising the Cy3 and Cy5 fluorescence intensity clustering in 2D Cartesian plots and re-centring the clusters. The refined genotype calls were scrutinised further before exporting genotype calls knowing that the parents and the lines of this population were doubled haploids and so any SNPs that failed to show only two- group clustering were not credible: SNPs with more than two call clustered were set to 'no-call'.

### Phenotyping of DH plants

### a) Choice of the isolates

The isolate used was obtained from the University of Alberta, AB, Canada. The University collected it from Leduc, Alberta Canada. It was single spored and named Leduc ss2 which was characterized as Pathotype 6 by Xue et. Al. (Xue et al. 2008. Isolation and variation of single spore isolates of Plasmodiophora brassicae from Canada, Plant Dis. 92:456-462, Strelkov et. el. 2006. Characterization of Plasmodiophora brassicae populations from Alberta, Canada. Can. J. Plant Pathol. 28:467-474).

### b) Phenotyping of the DH population

106 DH lines with a sufficient amount of seeds have been phenotyped for Clubroot disease resistance by inoculation with isolate Leduc ss2 and disease ratings on a 1 - 3 scale.

### Genetic map and QTL analysis

A total of 363 polymorphic SNP marker instances were observed over the individuals of the DH mapping population (1022 markers were monomorphic and 151 markers produced no calls). Additionally, in this DH1 (a doubled haploid population produced from the gametes of the F1 of a cross between two homozygous diploid parents) population, the Clubroot Resistance phenotype was scored as with the sensitive parent allele 'A' and the resistant parent allele 'B', allowing this score to be treated as though it was a marker. Genetic linkage mapping was performed using JoinMap^{®} 3.0 software (Van Ooijen, J.W. and R.E. Voorrips, 2001, JoinMap® 3.0 software for the calculation of genetic linkage maps. Plant Research International, Wageningen, Netherlands).

Linked marker genotype groupings were identified by examining the Lod- grouping tree results that ranged from Lod3.0 to Lod10. With groups of linked markers identified, ordering of the markers was performed using the default calculation options, with the 3rd round option and using the Kosambi mapping function.

### Additional markers added to rough mapping

96 additional KASP markers were genotyped on the rough mapping population. No recombination point was observed based on these additional markers. Based on the marker sequences the genomic region comprising the *CRL* clubroot resistance gene was identified.

### Example 2 - Fine mapping of Clubroot resistance using SNP marker analysis

An F2 population of 1000 individuals originating from the F1 cross between the resistant female parent and the susceptible male parent was obtained. 1000 F2 plants were genotyped together with the parents using 96 KASP markers located at the bottom of N02 using the Fluidigm platform. 74 markers were mapped. Two markers defining the border were identified (markers mBRS00013411 and mBRS00013511; see Table 2). 6 markers between the border gave no call in the susceptible parent, whereas they gave a call in the resistant parent (markers mBRS00013440, mBRS00154152, mBRS00013441, mBRS00013442, mBRS00161831, and mBRS00174679; see Table 2), indicating the absence of a region between the flanking markers in the susceptible parent. After finemapping the plotted region of interest (present in the resistant parent but absent in the susceptible parent) comprising the *CRL* gene has been defined as a 563 kb region.

Table 2. Markers linked to *CRL* clubroot resistance and susceptibility and primer combinations for detecting of these markers. R = Resistant line; S = Susceptible line. The polymorphic base detected by Primer 1 and indicated with R is the marker allele present in the resistant line *(CRL* clubroot resistance marker allele); the polymorphic base detected by Primer 2 and indicated with S is the marker allele present in the susceptible line *(CRL* clubroot susceptibility marker allele). None: the polymorphic base is not present in the resistant line and is not present in the susceptible line.

| Marker | Primers | Polymorphic base | Allele in R / S | SEQ ID NO: |
|---|---|---|---|---|
| mBRS00013411 | Primer 1 | C | R | 12 |
| | Primer 2 | T | S | 13 |
| | common primer | - | Common | 14 |
| mBRS00013412 | Primer 1 | C | R | 15 |
| | Primer 2 | T | S | 16 |
| | common primer | - | Common | 17 |
| mBRS00013414 | Primer 1 | C | R | 18 |
| | Primer 2 | G | S | 19 |
| | common primer | - | Common | 20 |
| mBRS00013415 | Primer 1 | T | R | 21 |
| | Primer 2 | G | S | 22 |
| | common primer | - | Common | 23 |
| mBRS00003251 | Primer 1 | T | R | 24 |
| | Primer 2 | C | S | 25 |
| | common primer | - | Common | 26 |
| mBRS00013416 | Primer 1 | A | R | 27 |
| | Primer 2 | G | S | 28 |
| | common primer | - | Common | 29 |
| mBRS00013417 | Primer 1 | A | R | 30 |
| | Primer 2 | T | S | 31 |
| | common primer | - | Common | 32 |
| mBRS00143666 | Primer 1 | G | R | 33 |
| | Primer 2 | A | S | 34 |
| | common primer | - | Common | 35 |
| mBRS00013418 | Primer 1 | T | R | 36 |
| | Primer 2 | C | S | 37 |
| | common primer | - | Common | 38 |
| mBRS00013421 | Primer 1 | T | R | 39 |
| | Primer 2 | A | S | 40 |
| | common primer | - | Common | 41 |
| mBRS00006010 | Primer 1 | T | R | 42 |
| | Primer 2 | G | S | 43 |
| | common primer | - | Common | 44 |
| mBRS00139820 | Primer 1 | A | R | 45 |
| | Primer 2 | C | S | 46 |
| | common primer | - | Common | 47 |
| mBRS00013422 | Primer 1 | A | R | 48 |
| | Primer 2 | G | S | 49 |
| | common primer | - | Common | 50 |
| mBRS00152839 | Primer 1 | A | R | 51 |
| | Primer 2 | T | S | 52 |
| | common primer | - | Common | 53 |
| mBRS00007859 | Primer 1 | G | R | 54 |
| | Primer 2 | A | S | 55 |
| | common primer | - | Common | 56 |
| mBRS00013424 | Primer 1 | A | R | 57 |
| | Primer 2 | G | S | 58 |
| | common primer | - | Common | 59 |
| mBRS00007860 | Primer 1 | T | R | 60 |
| | Primer 2 | C | S | 61 |
| | common primer | - | Common | 62 |
| mBRS00013426 | Primer 1 | C | R | 63 |
| | Primer 2 | T | S | 64 |
| | common primer | - | Common | 65 |
| mBRS00013427 | Primer 1 | G | R | 66 |
| | Primer 2 | A | S | 67 |
| | common primer | - | Common | 68 |
| mBRS00013428 | Primer 1 | A | R | 69 |
| | Primer 2 | T | S | 70 |
| | common primer | - | Common | 71 |
| mBRS00159860 | Primer 1 | A | R | 72 |
| | Primer 2 | G | S | 73 |
| | common primer | - | Common | 74 |
| mBRS00013429 | Primer 1 | T | R | 75 |
| | Primer 2 | C | S | 76 |
| | common primer | - | Common | 77 |
| mBRS00013431 | Primer 1 | T | R | 78 |
| | Primer 2 | C | S | 79 |
| | common primer | - | Common | 80 |
| mBRS00013432 | Primer 1 | A | R | 81 |
| | Primer 2 | C | S | 82 |
| | common primer | - | Common | 83 |
| mBRS00013433 | Primer 1 | T | R | 84 |
| | Primer 2 | C | S | 85 |
| | common primer | - | Common | 86 |
| mBRS01078993 | Primer 1 | A | R | 87 |
| | Primer 2 | G | S | 88 |
| | common primer | - | Common | 89 |
| mBRS00144440 | Primer 1 | T | R | 90 |
| | Primer 2 | A | S | 91 |
| | common primer | - | Common | 92 |
| mBRS00013434 | Primer 1 | G | R | 93 |
| | Primer 2 | C | S | 94 |
| | common primer | - | Common | 95 |
| mBRS00013435 | Primer 1 | T | R | 96 |
| | Primer 2 | A | None | 97 |
| | common primer | - | Common | 98 |
| mBRS00013436 | Primer 1 | T | R | 99 |
| | Primer 2 | A | S | 100 |
| | common primer | - | Common | 101 |
| mBRS00013437 | Primer 1 | T | R | 102 |
| | Primer 2 | A | S | 103 |
| | common primer | - | Common | 104 |
| mBRS00013438 | Primer 1 | T | R | 105 |
| | Primer 2 | G | S | 106 |
| | common primer | - | Common | 107 |
| mBRS00147882 | Primer 1 | T | R | 108 |
| | Primer 2 | C | S | 109 |
| | common primer | - | Common | 110 |
| mBRS00158190 | Primer 1 | T | R | 111 |
| | Primer 2 | A | S | 112 |
| | common primer | - | Common | 113 |
| mBRS00175448 | Primer 1 | T | R | 114 |
| | Primer 2 | A | S | 115 |
| | common primer | - | Common | 116 |
| mBRS00013440 | Primer 1 | A | R | 117 |
| | Primer 2 | C | None | 118 |
| | common primer | - | Common | 119 |
| mBRS00154152 | Primer 1 | C | R | 120 |
| | Primer 2 | G | None | 121 |
| | common primer | - | Common | 122 |
| mBRS00013441 | Primer 1 | T | R | 123 |
| | Primer 2 | C | None | 124 |
| | common primer | - | Common | 125 |
| mBRS00013442 | Primer 1 | T | R | 126 |
| | Primer 2 | A | None | 127 |
| | common primer | - | Common | 128 |
| mBRS00161831 | Primer 1 | C | R | 129 |
| | Primer 2 | T | None | 130 |
| | common primer | - | Common | 131 |
| mBRS00013444 | Primer 1 | A | R | 132 |
| | Primer 2 | C | S | 133 |
| | common primer | - | Common | 134 |
| mBRS00174679 | Primer 1 | C | R | 135 |
| | Primer 2 | T | None | 136 |
| | common primer | - | Common | 137 |
| mBRS00013446 | Primer 1 | C | R | 138 |
| | Primer 2 | T | S | 139 |
| | common primer | - | Common | 140 |
| mBRS00013450 | Primer 1 | C | R | 141 |
| | Primer 2 | A | S | 142 |
| | common primer | - | Common | 143 |
| mBRS00013451 | Primer 1 | G | R | 144 |
| | Primer 2 | A | S | 145 |
| | common primer | - | Common | 146 |
| mBRS00159719 | Primer 1 | T | R | 147 |
| | Primer 2 | C | S | 148 |
| | common primer | - | Common | 149 |
| mBRS00011851 | Primer 1 | C | R | 150 |
| | Primer 2 | A | S | 151 |
| | common primer | - | Common | 152 |
| mBRS00013453 | Primer 1 | G | R | 153 |
| | Primer 2 | C | S | 154 |
| | common primer | - | Common | 155 |
| mBRS00013456 | Primer 1 | C | R | 156 |
| | Primer 2 | G | S | 157 |
| | common primer | - | Common | 158 |
| mBRS01079015 | Primer 1 | T | R | 159 |
| | Primer 2 | G | S | 160 |
| | common primer | - | Common | 161 |
| mBRS00013459 | Primer 1 | G | R | 162 |
| | Primer 2 | C | S | 163 |
| | common primer | - | Common | 164 |
| mBRS00153607 | Primer 1 | G | R | 165 |
| | Primer 2 | A | S | 166 |
| | common primer | - | Common | 167 |
| mBRS00013497 | Primer 1 | C | R | 168 |
| | Primer 2 | A | None | 169 |
| | common primer | - | Common | 170 |
| mBRS00013499 | Primer 1 | A | R | 171 |
| | Primer 2 | C | S | 172 |
| | common primer | - | Common | 173 |
| mBRS00013511 | Primer 1 | C | R | 174 |
| | Primer 2 | T | S | 175 |
| | common primer | - | Common | 176 |

### Example 3 - Identification of Clubroot resistance genes

### Identification of syntenic region in B. napus

The DNA sequence of the part of the pseudochromosome between the closest flanking markers of a *B. rapa* line, was blasted against the genome sequence scaffolds of the resistant female parent. The genome sequence between the flanking markers of the resistant female parent is shown in SEQ ID NO: 1. SEQ ID NO: 1 contains two putative genes with a CC-NBS-LRR domain or disease resistance classification *(CRL1* and *CRL2,* respectively), wherein four different splice variants exist for *CRL*1 (*CRL1*a, *CRL1b, CRL*1c*,* and *CRL*1d respectively). The cDNA sequences of the *CRL1a, CRL*1b, *CRL*1c*, CRL*1d ,and *CRL2* genes are shown in SEQ ID NO: 2, SEQ ID NO: 4, SEQ ID NO: 6, SEQ ID NO: 10, and SEQ ID NO: 8, respectively. The coding sequences of *CRL*1a, *CRL*1b, *CRL*1c*, CRL*1d , and *CRL2* are at nt 52-5343 of SEQ ID NO: 2, nt 52-5340 of SEQ ID NO: 4, nt 52-5361 of SEQ ID NO: 6, nt 1-5259 of SEQ ID NO: 10, and nt 220-2898 of SEQ ID NO: 8, respectively. The encoded CRL1a, CRL1b, CRL1c, CRL1d, and CRL2 proteins are shown in SEQ ID NO: 3, SEQ ID NO: 5, SEQ ID NO: 7, SEQ ID NO: 11, and SEQ ID NO: 9, respectively. The *CRL*1b splicing variant was most abundant, followed by the *CRL*1a splicing variant. The *CRL*1c and *CRL*1d splicing variants were the least abundant.

Seeds of *B. napus* comprising the *CRL* clubroot resistance locus have been deposited at the NCIMB (NCIMB Ltd, Ferguson Building, Craibstone Estate, Bucksburn, Aberdeen AB21 9YA, Scotland, UK) on 7 December 2015, under accession number NCIMB 42499 and on 22 July 2016, under accession number NCIMB 42610.

### Validation of clubroot R genes

Mutations in the *CRL1* and *CRL2* genes of *Brassica napus* identified in Example 3 were generated and identified as follows:
- 30,000 seeds from an elite spring oilseed rape breeding line (M0 seeds) were pre-imbibed for 2 h on wet filter paper in deionized or distilled water. Half of the seeds were exposed to 0.8% EMS and half to 1% EMS (Sigma: M0880) and incubated for 4 h.
- The mutagenized seeds (M1 seeds) were rinsed three times and dried in a fume hood overnight. 30,000 M1 plants were grown in soil and selfed to generate M2 seeds. M2 seeds were harvested for each individual M1 plant.
- Two times 4800 M2 plants, derived from different M1 plants, were grown and DNA samples were prepared from leaf samples of each individual M2 plant according to the CTAB method (Doyle and Doyle, 1987, Phytochemistry Bulletin 19:11-15).
- The DNA samples were screened for the presence of point mutations in the *CRL1* and *CRL2* genes that cause the introduction of STOP codons and another amino acid in the protein-encoding regions of the *CRL1* and *CRL2* genes, by direct sequencing by standard sequencing techniques (LGC) and analyzing the sequences for the presence of the point mutations using the NovoSNP software (VIB Antwerp).
- The mutant *CRL*1 and *CRL2* alleles as depicted in Table 3 were thus identified.

Table 3: STOP codon mutations in *CRL1* and *CRL2*

**Table 3a: mutations in CRL1**

| Plant name | Nt pos in SEQ ID NO:1 | Nt pos cDNA (SEQ ID NO) | AA pos. (SEQ ID NO) | WT → mut codon | WT → mut AA |
|---|---|---|---|---|---|
| CLUB301 | 33323 | 574 (2) | 175 (3) | CAA→TAA | Q→STOP |
| | | 574 (4) | 175 (4) | | |
| | | 574 (6) | 175 (5) | | |
| CLUB202 | 44587 | 3371 (2) | 1107 (3) | TGG→TAG | W→STOP |
| | | 3368 (4) | 1106 (5) | | |
| | | 3389 (6) | 1113 (7) | | |
| CLUB101 | 49414 | 4060 (2) | 1337 | CGA→TGA | R→STOP |
| | | 4057 (4) | 1336 | | |
| | | 4078 (6) | 1343 | | |

**Table 3b: mutations in CRL2**

| Plant name | Nt pos in SEQ ID NO:1 | Nt pos cDNA SEQ ID NO:6 | AA pos. SEQ ID NO:7 | WT → mut codon | WT → mut AA |
|---|---|---|---|---|---|
| CLUB401 | 3992 | 848 | 210 | TGG→TAG | W→STOP |

Plants comprising the CLUB301, CLUB202, CLUB101 or CLUB401 mutation were analyzed for loss of resistance to *Plasmodiophora brassicae* pathotype 6.

Briefly, between 15 and 30 plants were grown for each homozygous mutant and corresponding wild type segregant. Plants were inoculated with Pathotype6 and disease symptoms on roots were rated 32 days post inoculation using a 0-3 rating scale. A Disease Index (DI) was calculated:
0 = no galling
1 = a few small galls on 1/3 of the roots
2 = moderate galling on 1/3 to 2/3 of the roots
3 = severe galling on more than 2/3 of the roots
$DI \left(%\right) = \frac{\sum \left(n\times 0+n\times 1+n\times 2+n\times 3\right)}{N \times 3} \times 100 %$ Where Σ is the sum total; *n* is the number of plants in a class; *N* is the total number fo plants; and 0, 1, 2, and 3 are the symptom severity classes.

Each one of the four mutations lead to loss of resistance to *Plasmodiophora brassicae* pathotype 6. These results show that both the *CRL1* and the *CRL2* genes are required for clubroot resistance.

The *CRL*1a, *CRL1b, CRL*1c*, CRL*1d, and *CRL2* coding sequences are cloned under control of a constitutive 35S promoter, and under control of their native promoters in a T-DNA expression vector. The genomic region comprising the *CRL*1a, *CRL1b, CRL*1c, and *CRL*1d cDNAs, i.e. nt 32750 to 51049 of SEQ ID NO: 1, is also cloned under control of a constitutive 35S promoter and under control of its native promoter in a T-DNA expression vector. The genomic region comprising the *CRL1* and *CRL2* coding sequences of SEQ ID NO: 1 is cloned under the control of a in a T-DNA expression vector. All T-DNA expression vectors comprise a selectable marker. The resulting vectors are transformed in *Brassica napus* cv. Westar, which is susceptible to clubroot, using the hypocotyl transformation protocol essentially as described by De Block et al. (1989), Plant Physiol. 91: 694. The copy number of the transgene in the transgenic plant is determined by real time PCR on the bar gene. The transformed plants with comprising the *CRL1* and *CRL2* genes, or the *CRL*1a, *CRL*1b, *CRL*1c*,* or *CRL*1d, and *CRL2* coding sequences as transgene show increased clubroot resistance as compared to *Brassica napus* cv. Westar plants not comprising the *CRL1* and *CRL2* gene as transgene.

### Example 4 - Transfer of CRL1 and CRL2 into other Brassicaceae lines

The *CRL1* and *CRL2* genes are transferred into Brassicaceae breeding lines by the following method: A plant containing the *CRL1* and *CRL2* genes (donor plant), is crossed with a Brassicaceae line (elite parent / recurrent parent) or variety lacking the *CRL1* and *CRL2* genes. The following introgression scheme is used, wherein the presence of the *CRL1* and *CRL2* genes is indicated with *CRL,* and the absence of the *CRL* gene is indicated with -:

| | | | | |
|---|---|---|---|---|
| Initial cross: | *CRL* /*CRL* (donor plant) | | X | - / - (elite parent) |
| F1 plant: | *CRL* / *-* | | | |
| BC1 cross: | *CRL* /*-* | X | - / - (recurrent parent) | |
| BC1 plants: | 50% *CRL* / - and 50% - / - | | | |

The 50% *CRL* /- are selected using molecular markers (e.g. AFLP, PCR, Invader^{™}, KASP, such as the markers of Table 2, and the like) for the presence of the *CRL* gene.
Further backcrosses can be performed. Upon one or more steps of backcrossing (BCx), backcrossed plants heterozygous for *CRL* are selfed:

| | | | |
|---|---|---|---|
| BCx S1 cross: | *CRL* /- | X | *CRL* /*-* |
| BCx S1 plants: | *25% CRL* / *CRL* and 50% *CRL* / - and 25% - / - | | |

Plants containing *CRL* are selected using molecular markers, such as the markers of Table 2, for the linked with the *CRL* gene. Individual BCx S1 plants that are homozygous for *CRL (CRL* / *CRL)* are selected using molecular markers linked with *CRL,* such as the markers of Table 2. These plants can then be used for seed production.

## Claims

1. A method to determine the presence or absence of a *CRL* clubroot resistance locus in a biological sample of a *Brassica napus* plant comprising providing genomic DNA from said biological sample, and analyzing said DNA for the presence of at least one molecular marker, wherein the at least one molecular marker is linked to said *CRL* clubroot resistance locus, wherein
said *CRL* clubroot resistance locus comprises the *CRL1* and *CRL2* clubroot resistance genes, wherein
a) said *CRL1* clubroot resistance gene comprises a nucleotide sequence
- having at least 90% sequence identity to nt 32750-51049 of SEQ ID NO: 1;
- having a coding sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or
- encoding a protein having at least 90% sequence identity to SEQ ID NO: 3, SEQ ID NO: 5, SEQ ID NO: 7, or SEQ ID NO: 11; and
b) said *CRL2* clubroot resistance gene comprises a nucleotide sequence
- having a coding sequence having at least 90% sequence identity to nt 220-2898 of SEQ ID NO: 8; or
- encoding a protein having at least 90% sequence identity to SEQ ID NO: 9
wherein said marker is located in a marker interval between and including markers mBRS00013411 and mBRS00013511, wherein marker mBRS00013411 contains the polymorphic base detected by the primer having the sequence of SEQ ID NO: 12, and wherein marker mBRS00013511 contains the polymorphic base detected by the primer having the sequence of SEQ ID NO: 174.

2. A kit for the detection of a *CRL* clubroot resistance locus as described in claim 1 in DNA samples of a *Brassica napus* plant, wherein said kit comprises at least one primer or probe which specifically recognizes a molecular marker linked to said *CRL* clubroot resistance locus
wherein said marker is located in a marker interval between and including markers mBRS00013411 and mBRS00013511, wherein marker mBRS00013411 contains the polymorphic base detected by the primer having the sequence of SEQ ID NO: 12, and wherein marker mBRS00013511 contains the polymorphic base detected by the primer having the sequence of SEQ ID NO: 174.

3. The method or the kit according to claim 1 or 2, wherein said marker is selected from the marker containing the polymorphic base detected by the primer having the sequence of SEQ ID NO: 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171, and 174.

4. Use of a molecular marker linked to the *CRL* clubroot resistance locus as described in claim 1 or 3 for determining the presence or absence of said *CRL* clubroot resistance locus in *Brassica napus* plants.

5. Use of the sequence of any one of SEQ ID NOs: 1, 2, 4, 6, 8, and 10 for determining the presence or absence of a *CRL* clubroot resistance locus in Brassicaceae plants.

6. Use of the sequence of any one of SEQ ID NOs: 1, 2, 4, 6, 8, and 10 for developing a marker for determining the presence or absence of a *CRL* clubroot resistance locus in Brassicaceae plants.

7. A Brassicaceae plant or plant cell comprising a *CRL1* and a *CRL2* clubroot resistance gene as transgene, wherein
a) said *CRL1* clubroot resistance gene
- comprises a coding sequence having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or
- encodes a protein having an amino acid sequence having at least 90% sequence identity to SEQ ID NO: 3, SEQ ID NO: 5, SEQ ID NO: 7, or SEQ ID NO: 11; and
b) said *CRL2* clubroot resistance gene
- comprises a coding sequence having at least 90% sequence identity to nt 220-2898 of SEQ ID NO: 8; or
- encodes a protein having an amino acid sequence having at least 90% sequence identity to SEQ ID NO: 9.

8. A method for increasing clubroot resistance in a Brassicaceae plant, comprising:
a) Introducing by transformation a *CRL1* and a *CRL2* clubroot resistance gene as described in claim 7 as transgene, to a Brassicaceae plant cell, to create transgenic cells; and
b) regenerating transgenic plants from said transgenic cells.

9. A Brassicaceae plant or plant cell according to claim 7, comprising said *CRL1* and *CRL2* clubroot resistance genes, further comprising at least one other disease resistance gene, said other disease resistance gene selected from the group consisting of a clubroot resistance gene, a blackleg resistance gene, a Sclerotinia resistance gene, a Verticillium resistance gene, a Fusarium resistance gene, an Aster Yellows resistance gene, an Alternaria resistance gene, and a Grey Stem resistance gene.

10. The plant or plant cell according to claim 9, wherein said other disease resistance gene is a transgene which is genetically linked with said *CRL* clubroot resistance gene or said clubroot resistance locus.

11. The Brassicaceae plant or plant cell according to any one of claims 7, 9 and 10, which is selected from the group consisting of *Brassica napus, Brassica juncea, Brassica oleracea, Brassica rapa, Brassica nigra* and *Brassica carinata.*

12. Seeds of the plant of any one of claims 7 and 9 to 11, said seeds comprising said *CRL*1 and *CRL2* clubroot resistance genes.

13. A chimeric gene comprising the following operably linked elements:
a) a plant-expressible promoter;
b) a DNA sequence coding for a CRL1 or for a CRL2 protein; and
c) optionally, a transcription termination and polyadenylation region functional in plant cells,
wherein
a) said DNA sequence coding for a CRL1 protein comprises a nucleotide sequence
- having at least 90% sequence identity to nt 52-5343 of SEQ ID NO: 2, nt 52-5340 of SEQ ID NO: 4, nt 52-5361 of SEQ ID NO: 6, or to SEQ ID NO: 10; or
- encoding a protein having an amino acid sequence having at least 90% sequence identity to SEQ ID NO: 3, SEQ ID NO: 5, SEQ ID NO: 7, or SEQ ID NO: 11; and
b) said DNA sequence coding for a CRL2 protein comprises a nucleotide sequence
- having at least 90% sequence identity to 220-2898 of SEQ ID NO: 8; or
- encoding a protein having an amino acid sequence having at least 90% sequence identity to SEQ ID NO: 9.

14. Use of the plant or plant cell of any one of claims 7 and 9 to 11 to produce oilseed rape oil or an oilseed rape seed cake, or a seed, or a crop of oilseed rape.

## Patentansprüche

1. Verfahren zum Bestimmen des Vorhandenseins oder Nichtvorhandenseins eines CRL-Kohlhernie-Resistenzlokus in einer biologischen Probe einer *Brassica napus*-Pflanze, umfassend das Bereitstellen genomischer DNA aus der genannten biologischen Probe und das Analysieren der genannten DNA auf das Vorhandensein mindestens eines molekularen Markers, wobei der mindestens eine molekulare Marker mit dem genannten CRL-Kohlhernie-Resistenzlokus gekoppelt ist, wobei
der genannte CRL-Kohlhernie-Resistenzlokus die Kohlhernie-Resistenzgene CRL1 und CRL2 umfasst, wobei
a) das genannte Kohlhernie-Resistenzgen CRL1 eine Nukleotidsequenz umfasst,
• die mindestens 90% Sequenzidentität zu nt 32750-51049 von SEQ ID NO: 1 aufweist;
• die eine kodierende Sequenz aufweist, die mindestens 90% Sequenzidentität zu nt 52-5343 von SEQ ID NO: 2, nt 52-5340 von SEQ ID NO: 4, nt 52-5361 von SEQ ID NO: 6 oder zu SEQ ID NO: 10 aufweist; oder
• die ein Protein kodiert, das mindestens 90% Sequenzidentität zu SEQ ID NO: 3, SEQ ID NO: 5, SEQ ID NO: 7 oder SEQ ID NO: 11 aufweist; und
b) das genannte Kohlhernie-Resistenzgen CRL2 eine Nukleotidsequenz umfasst,
• die eine kodierende Sequenz aufweist, die mindestens 90% Sequenzidentität zu nt 220-2898 von SEQ ID NO: 8 aufweist; oder
• die ein Protein kodiert, das mindestens 90% Sequenzidentität zu SEQ ID NO: 9 aufweist,
wobei sich der genannte Marker in einem Markerintervall zwischen den Markern mBRS00013411 und mBRS00013511, einschließlich dieser Marker, befindet, wobei der Marker mBRS00013411 die polymorphe Base enthält, die durch den Primer mit der Sequenz von SEQ ID NO: 12 detektiert wird, und wobei der Marker mBRS00013511 die polymorphe Base enthält, die durch den Primer mit der Sequenz von SEQ ID NO: 174 detektiert wird.

2. Kit zum Nachweis eines CRL-Kohlhernie-Resistenzlokus gemäß Anspruch 1 in DNA-Proben einer *Brassica napus*-Pflanze, wobei das Kit mindestens einen Primer oder eine Sonde umfasst, der bzw. die spezifisch einen mit dem genannten CRL-Kohlhernie-Resistenzlokus gekoppelten molekularen Marker erkennt,
wobei sich der genannte Marker in einem Markerintervall zwischen den Markern mBRS00013411 und mBRS00013511, einschließlich dieser Marker, befindet, wobei der Marker mBRS00013411 die polymorphe Base enthält, die durch den Primer mit der Sequenz von SEQ ID NO: 12 detektiert wird, und wobei der Marker mBRS00013511 die polymorphe Base enthält, die durch den Primer mit der Sequenz von SEQ ID NO: 174 detektiert wird.

3. Verfahren oder Kit nach Anspruch 1 oder 2, wobei der genannte Marker aus dem Marker ausgewählt ist, der die polymorphe Base enthält, die durch den Primer mit der Sequenz von SEQ ID NO: 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171 und 174 detektiert wird.

4. Verwendung eines mit dem CRL-Kohlhernie-Resistenzlokus gekoppelten molekularen Markers gemäß Anspruch 1 oder 3 zur Bestimmung des Vorhandenseins oder Nichtvorhandenseins des genannten CRL-Kohlhernie-Resistenzlokus in *Brassica napus-*Pflanzen.

5. Verwendung der Sequenz einer beliebigen der SEQ ID NOs: 1, 2, 4, 6, 8 und 10 zur Bestimmung des Vorhandenseins oder Nichtvorhandenseins eines CRL-Kohlhernie-Resistenzlokus in Pflanzen der Familie Brassicaceae.

6. Verwendung der Sequenz einer beliebigen der SEQ ID NOs: 1, 2, 4, 6, 8 und 10 zur Entwicklung eines Markers zur Bestimmung des Vorhandenseins oder Nichtvorhandenseins eines CRL-Kohlhernie-Resistenzlokus in Pflanzen der Familie Brassicaceae.

7. Brassicaceae-Pflanze oder Pflanzenzelle, die ein CRL1- und ein CRL2-Kohlhernie-Resistenzgen als Transgen umfasst, wobei
a) das genannte CRL1-Kohlhernie-Resistenzgen
• eine kodierende Sequenz umfasst, die mindestens 90% Sequenzidentität zu nt 52-5343 von SEQ ID NO: 2, nt 52-5340 von SEQ ID NO: 4, nt 52-5361 von SEQ ID NO: 6 oder zu SEQ ID NO: 10 aufweist; oder
• ein Protein kodiert, dessen Aminosäuresequenz mindestens 90% Sequenzidentität zu SEQ ID NO: 3, SEQ ID NO: 5, SEQ ID NO: 7 oder SEQ ID NO: 11 aufweist; und
b) das genannte CRL2-Kohlhernie-Resistenzgen
• eine kodierende Sequenz umfasst, die mindestens 90% Sequenzidentität zu nt 220-2898 von SEQ ID NO: 8 aufweist; oder
• ein Protein kodiert, dessen Aminosäuresequenz mindestens 90% Sequenzidentität zu SEQ ID NO: 9 aufweist.

8. Verfahren zur Erhöhung der Kohlhernie-Resistenz in einer Brassicaceae-Pflanze, umfassend:
a) das Einbringen eines CRL1- und eines CRL2-Kohlhernie-Resistenzgens gemäß Anspruch 7 als Transgen durch Transformation in eine Brassicaceae-Pflanzenzelle zur Erzeugung transgener Zellen; und
b) die Regeneration transgener Pflanzen aus den genannten transgenen Zellen.

9. Brassicaceae-Pflanze oder Pflanzenzelle nach Anspruch 7, die die genannten CRL1- und CRL2-Kohlhernie-Resistenzgene umfasst und ferner mindestens ein weiteres Krankheitsresistenzgen umfasst, wobei das weitere Krankheitsresistenzgen ausgewählt ist aus der Gruppe bestehend aus einem Kohlhernie-Resistenzgen, einem Phoma-Resistenzgen (Blackleg), einem Sclerotinia-Resistenzgen, einem Verticillium-Resistenzgen, einem Fusarium-Resistenzgen, einem Resistenzgen gegen Aster Yellows, einem Alternaria-Resistenzgen und einem Resistenzgen gegen Graustängeligkeit (Grey Stem).

10. Pflanze oder Pflanzenzelle nach Anspruch 9, wobei das weitere Krankheitsresistenzgen ein Transgen ist, das genetisch mit dem genannten CRL-Kohlhernie-Resistenzgen oder dem genannten Kohlhernie-Resistenzlokus gekoppelt ist.

11. Brassicaceae-Pflanze oder Pflanzenzelle nach einem der Ansprüche 7, 9 und 10, ausgewählt aus der Gruppe bestehend aus *Brassica napus*, *Brassica juncea*, *Brassica oleracea*, *Brassica rapa*, *Brassica nigra* und *Brassica carinata*.

12. Samen der Pflanze nach einem der Ansprüche 7 und 9 bis 11, wobei die Samen die genannten CRL1- und CRL2-Kohlhernie-Resistenzgene umfassen.

13. Chimäres Gen, umfassend die folgenden funktionsgekoppelten Elemente:
a) einen in Pflanzen exprimierbaren Promotor;
b) eine DNA-Sequenz, die für ein CRL1- oder ein CRL2-Protein kodiert; und
c) optional eine in Pflanzenzellen funktionelle Transkriptions-Terminations- und Polyadenylierungsregion,
wobei
a) die genannte DNA-Sequenz, die für ein CRL1-Protein kodiert, eine Nukleotidsequenz umfasst,
• die mindestens 90% Sequenzidentität zu nt 52-5343 von SEQ ID NO: 2, nt 52-5340 von SEQ ID NO: 4, nt 52-5361 von SEQ ID NO: 6 oder zu SEQ ID NO: 10 aufweist; oder
• die ein Protein kodiert, dessen Aminosäuresequenz mindestens 90% Sequenzidentität zu SEQ ID NO: 3, SEQ ID NO: 5, SEQ ID NO: 7 oder SEQ ID NO: 11 aufweist; und
b) die genannte DNA-Sequenz, die für ein CRL2-Protein kodiert, eine Nukleotidsequenz umfasst,
• die mindestens 90% Sequenzidentität zu nt 220-2898 von SEQ ID NO: 8 aufweist; oder
• die ein Protein kodiert, dessen Aminosäuresequenz mindestens 90% Sequenzidentität zu SEQ ID NO: 9 aufweist.

14. Verwendung der Pflanze oder Pflanzenzelle nach einem der Ansprüche 7 und 9 bis 11 zur Herstellung von Rapsöl oder Rapspresskuchen, von Saatgut oder eines Rapsbestands.

## Revendications

1. Procédé permettant de déterminer la présence ou l'absence d'un locus de résistance à la hernie des crucifères CRL dans un échantillon biologique d'une plante de Brassica napus, comprenant la fourniture d'ADN génomique provenant dudit échantillon biologique, et l'analyse dudit ADN afin de détecter la présence d'au moins un marqueur moléculaire, ledit au moins un marqueur moléculaire étant lié audit locus de résistance à la hernie des crucifères CRL, dans lequel:
ledit locus de résistance à la hernie des crucifères CRL comprend les gènes de résistance à la hernie des crucifères CRL1 et CRL2, et dans lequel:
a) ledit gène de résistance à la hernie des crucifères CRL1 comprend une séquence nucléotidique:
• présentant au moins 90% d'identité de séquence avec les nt 32750-51049 de la SEQ ID NO: 1;
• présentant une séquence codante ayant au moins 90% d'identité de séquence avec les nt 52-5343 de la SEQ ID NO: 2, les nt 52-5340 de la SEQ ID NO: 4, les nt 52-5361 de la SEQ ID NO: 6, ou avec la SEQ ID NO: 10; ou
• codant une protéine présentant au moins 90% d'identité de séquence avec la SEQ ID NO: 3, la SEQ ID NO: 5, la SEQ ID NO: 7 ou la SEQ ID NO: 11; et
b) ledit gène de résistance à la hernie des crucifères CRL2 comprend une séquence nucléotidique:
• présentant une séquence codante ayant au moins 90% d'identité de séquence avec les nt 220-2898 de la SEQ ID NO: 8; ou
• codant une protéine présentant au moins 90% d'identité de séquence avec la SEQ ID NO: 9;
dans lequel ledit marqueur est situé dans un intervalle de marqueurs compris entre les marqueurs mBRS00013411 et mBRS00013511, inclusivement, le marqueur mBRS00013411 contenant la base polymorphe détectée par l'amorce ayant la séquence de la SEQ ID NO: 12, et le marqueur mBRS00013511 contenant la base polymorphe détectée par l'amorce ayant la séquence de la SEQ ID NO: 174.

2. Kit destiné à la détection d'un locus de résistance à la hernie des crucifères CRL tel que décrit dans la revendication 1 dans des échantillons d'ADN d'une plante de Brassica napus, ledit kit comprenant au moins une amorce ou une sonde reconnaissant spécifiquement un marqueur moléculaire lié audit locus de résistance à la hernie des crucifères CRL,
dans lequel ledit marqueur est situé dans un intervalle de marqueurs compris entre les marqueurs mBRS00013411 et mBRS00013511, inclusivement, le marqueur mBRS00013411 contenant la base polymorphe détectée par l'amorce ayant la séquence de la SEQ ID NO: 12, et le marqueur mBRS00013511 contenant la base polymorphe détectée par l'amorce ayant la séquence de la SEQ ID NO: 174.

3. Procédé ou kit selon la revendication 1 ou 2, dans lequel ledit marqueur est choisi parmi le marqueur contenant la base polymorphe détectée par l'amorce ayant la séquence de la SEQ ID NO: 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171 et 174.

4. Utilisation d'un marqueur moléculaire lié au locus de résistance à la hernie des crucifères CRL tel que décrit dans la revendication 1 ou 3 pour déterminer la présence ou l'absence dudit locus de résistance à la hernie des crucifères CRL chez des plantes de Brassica napus.

5. Utilisation de la séquence de l'une quelconque des SEQ ID NO: 1, 2, 4, 6, 8 et 10 pour déterminer la présence ou l'absence d'un locus de résistance à la hernie des crucifères CRL chez des plantes appartenant à la famille des Brassicaceae.

6. Utilisation de la séquence de l'une quelconque des SEQ ID NO: 1, 2, 4, 6, 8 et 10 pour développer un marqueur permettant de déterminer la présence ou l'absence d'un locus de résistance à la hernie des crucifères CRL chez des plantes appartenant à la famille des Brassicaceae.

7. Plante ou cellule végétale de Brassicaceae comprenant, sous forme de transgène, un gène de résistance à la hernie des crucifères CRL1 et un gène de résistance à la hernie des crucifères CRL2, dans laquelle:
a) ledit gène de résistance à la hernie des crucifères CRL1:
• comprend une séquence codante ayant au moins 90% d'identité de séquence avec les nt 52-5343 de la SEQ ID NO: 2, les nt 52-5340 de la SEQ ID NO: 4, les nt 52-5361 de la SEQ ID NO: 6, ou avec la SEQ ID NO: 10; ou
• code une protéine présentant une séquence d'acides aminés ayant au moins 90% d'identité de séquence avec la SEQ ID NO: 3, la SEQ ID NO: 5, la SEQ ID NO: 7 ou la SEQ ID NO: 11; et
b) ledit gène de résistance à la hernie des crucifères CRL2:
• comprend une séquence codante ayant au moins 90% d'identité de séquence avec les nt 220-2898 de la SEQ ID NO: 8; ou
• code une protéine présentant une séquence d'acides aminés ayant au moins 90% d'identité de séquence avec la SEQ ID NO: 9.

8. Procédé visant à augmenter la résistance à la hernie des crucifères chez une plante de Brassicaceae, comprenant:
a) l'introduction, par transformation, d'un gène de résistance à la hernie des crucifères CRL1 et d'un gène de résistance à la hernie des crucifères CRL2 tels que décrits dans la revendication 7, sous forme de transgène, dans une cellule végétale de Brassicaceae, afin de créer des cellules transgéniques; et
b) la régénération de plantes transgéniques à partir desdites cellules transgéniques.

9. Plante ou cellule végétale de Brassicaceae selon la revendication 7, comprenant lesdits gènes de résistance à la hernie des crucifères CRL1 et CRL2, comprenant en outre au moins un autre gène de résistance à une maladie, ledit autre gène de résistance à une maladie étant choisi dans le groupe constitué d'un gène de résistance à la hernie des crucifères, d'un gène de résistance au phoma (blackleg), d'un gène de résistance à la sclérotiniose, d'un gène de résistance au Verticillium, d'un gène de résistance au Fusarium, d'un gène de résistance à la jaunisse de l'aster (Aster Yellows), d'un gène de résistance à l'Alternaria et d'un gène de résistance à la maladie de la tige grise (Grey Stem).

10. Plante ou cellule végétale selon la revendication 9, dans laquelle ledit autre gène de résistance à une maladie est un transgène génétiquement lié audit gène de résistance à la hernie des crucifères CRL ou audit locus de résistance à la hernie des crucifères.

11. Plante ou cellule végétale de Brassicaceae selon l'une quelconque des revendications 7, 9 et 10, choisie dans le groupe constitué de Brassica napus, Brassica juncea, Brassica oleracea, Brassica rapa, Brassica nigra et Brassica carinata.

12. Graines de la plante selon l'une quelconque des revendications 7 et 9 à 11, lesdites graines comprenant lesdits gènes de résistance à la hernie des crucifères CRL1 et CRL2.

13. Gène chimérique comprenant les éléments suivants liés fonctionnellement
a) un promoteur exprimable dans les plantes;
b) une séquence d'ADN codant pour une protéine CRL1 ou pour une protéine CRL2; et
c) facultativement, une région de terminaison de transcription et de polyadénylation fonctionnelle dans les cellules végétales,
dans lequel:
a) ladite séquence d'ADN codant pour une protéine CRL1 comprend une séquence nucléotidique:
• présentant au moins 90% d'identité de séquence avec les nt 52-5343 de la SEQ ID NO: 2, les nt 52-5340 de la SEQ ID NO: 4, les nt 52-5361 de la SEQ ID NO: 6, ou avec la SEQ ID NO: 10; ou
• codant une protéine présentant une séquence d'acides aminés ayant au moins 90% d'identité de séquence avec la SEQ ID NO: 3, la SEQ ID NO: 5, la SEQ ID NO: 7 ou la SEQ ID NO: 11; et
b) ladite séquence d'ADN codant pour une protéine CRL2 comprend une séquence nucléotidique:
• présentant au moins 90% d'identité de séquence avec les nt 220-2898 de la SEQ ID NO: 8; ou
• codant une protéine présentant une séquence d'acides aminés ayant au moins 90% d'identité de séquence avec la SEQ ID NO: 9.

14. Utilisation de la plante ou de la cellule végétale selon l'une quelconque des revendications 7 et 9 à 11 pour produire de l'huile de colza, un tourteau de colza, une semence ou une culture de colza.
